# EUROPÄISCHE PATENTSCHRIFT

(11) **EP 2 692 377 B1**
(45) Veröffentlichungstag und Bekanntmachung des Hinweises auf die Patenterteilung: **06.11.2019**
(21) Anmeldenummer: 12178916.8
(22) Anmeldetag: 01.08.2012
(51) Int. Cl.: A61M 5/20, A61M 5/31, A61M 5/315

(54) **Injektionsvorrichtung mit Dosisanzeige zur Signalisierung des Endes der Injektion**
Injection device with dose display element for signalling end of injection
Dispositif d'injection avec élément d'affichage de dose pour signaler fin d'injection

(43) Veröffentlichungstag der Anmeldung: 05.02.2014
(73) Patentinhaber: TecPharma Licensing AG, 3401 Burgdorf (CH)
(72) Erfinder: Streit, Ursina, 3322 Schönbühl (CH); Schenker, Susanne, 4900 Langenthal (CH)

(56) Entgegenhaltungen:
- WO-A1-2006/079481
- WO-A1-2009/039851
- WO-A1-2010/023303
- WO-A2-2012/032411
- CH-A1- 700 404
- DE-A1- 19 819 409

## Beschreibung

Die Erfindung betrifft eine Antriebs- und Dosiervorrichtung für eine Injektionsvorrichtung zur Verabreichung eines flüssigen Produkts, insbesondere eines Medikaments, wie zum Beispiel Insulin. Mit der Antriebs- und Dosiervorrichtung ist eine zu verabreichende Produktdosis einstellbar und vorzugsweise mittels mehrerer Einzelausschüttungen ausschüttbar, wobei die Schritte Dosieren und Ausschütten mehrfach wiederholt werden können. Die Erfindung betrifft somit auch eine Injektionsvorrichtung mit einer solchen Antriebs- und Dosiervorrichtung. Die Antriebs- und Dosiervorrichtung weist eine insbesondere mechanisch wirkende Dosisanzeige auf, an der die zu verabreichende Dosis ablesbar ist.

Aus dem Stand der Technik insbesondere der WO 2009/105909A1 ist eine Injektionsvorrichtung bekannt, die ein Gehäuse aufweist, in dem eine Dosisanzeigehülse angeordnet ist. Am hinteren Ende des Gehäuses ist ein in Bezug auf das Gehäuse drehbarer und axialfester Dosierknopf angeordnet. Durch Drehung des Dosierknopfs wird die Dosisanzeigetrommel an einem vom Gehäuse gebildeten Gewinde entlang geschraubt. Durch ein Fenster des Gehäuses ist die eingestellte Produktdosis ablesbar. Durch Betätigung eines Betätigungsknopfs ebenfalls am hinteren Ende des Gehäuses wird eine vorgespannte Antriebsfeder freigegeben, welche eine Kolbenstange für die Produktausschüttung antreibt und gleichzeitig die Dosisanzeigetrommel proportional zu der ausgeschütteten Produktmenge zurückdreht, wobei die in dem Fenster angezeigten Dosiswerte herunterzählen bzw. in Richtung des Dosiswerts Null zurücklaufen.

Diese Vorrichtung lässt vorteilhaft zu, dass bei einer Unterbrechung der Dosisausschüttung die noch auszuschüttende Produktmenge der teilweise ausgeschütteten Dosis in dem Fenster ablesbar ist. Der Verwender der Injektionsvorrichtung kann das Ende der Produktausschüttung dadurch erkennen, dass die Dosisanzeige stehen bleibt und in dem Fenster der Wert Null ablesbar ist.

Es gibt jedoch Patienten, die eine Signalisierung durch das Nichtvorhandensein einer Bewegung nicht sicher wahrnehmen können. Sondern eher eine aktive Signalisierung benötigen.

Weitere einschlägige Vorrichtungen sind in den Dokumenten WO 2006/079481 A1, WO 2009/039851 A1, WO 2012/032411 A2, CH 700 404 A1 und DE 198 19 408 A1 angegeben.

Es ist eine Aufgabe der Erfindung, eine Antriebs- und Dosiervorrichtung für eine Injektionsvorrichtung anzugeben, welche dem Verwender aktiv signalisiert, dass die Produktausschüttung abgeschlossen oder nahezu abgeschlossen ist.

Die Aufgabe wird mit dem Gegenstand des Anspruchs 1 gelöst. Vorteilhafte Weiterentwicklungen ergeben sich aus den abhängigen Ansprüchen, der Beschreibung und den Figuren.

Die Erfindung geht von einem Antriebsmechanismus, insbesondere einer Antriebs- und Dosiervorrichtung für eine Injektionsvorrichtung zum Verabreichen eines flüssigen Medikaments oder Produkts aus. Die Antriebs- und Dosiervorrichtung weist vorteilhaft ein Gehäuse auf. Das Gehäuse ist vorzugsweise hülsenförmig und/oder länglich ausgebildet. Das Gehäuse kann sich zum Beispiel entlang seiner Längsachse erstrecken.

Das Gehäuse kann optional einen Produktbehälter aufnehmen oder selbst den Produktbehälter bilden. Das Gehäuse kann ein- oder mehrteilig sein. Zum Beispiel kann das Gehäuse einen proximalen Gehäuseteil bilden, der die Antriebs- und Dosiervorrichtung umfasst oder aufweist. Das Gehäuse kann ferner einen Produktbehälterhalter aufweisen, der einen Produktbehälter, wie zum Beispiel eine Karpule, aufnimmt und mit dem Gehäuse oder dem proximalen Gehäuseteil verbunden ist. Diese Verbindung kann derart sein, dass der Produktbehälter und das Gehäuse oder der proximale Gehäuseteil nach dem Verbinden unlösbar, das heißt nur durch irreversible Zerstörung von Verbindungselementen voneinander lösbar sind. Eine solche Lösung ist insbesondere bei Einweginjektionsvorrichtungen von Vorteil, die nachdem das in dem Produktbehälter enthaltene Produkt vollständig ausgeschüttet ist, als Ganzes entsorgt werden. Alternativ kann der Produktbehälterhalter auch lösbar an dem Gehäuse befestigt sein, wodurch es möglich ist, wenngleich auch weniger bevorzugt, die Antriebs- und Dosiervorrichtung gegebenenfalls mehrfach zu verwenden, das heißt einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann zum Beispiel eine Zeigeeinrichtung, insbesondere ein Fenster aufweisen, mittels der oder durch welches eine aktuell eingestellte Dosis, vorzugsweise von einer Skala eines Dosiseinstellelements, ablesbar ist.

Die Antriebs- und Dosiervorrichtung umfasst ein Dosisanzeigeelement, über dessen Umfang eine Dosisskala angeordnet ist. Das Dosisanzeigeelement kann zum Beispiel im Querschnitt ringförmig sein. Das Dosisanzeigeelement kann zum Beispiel eine Dosisanzeigetrommel oder ein Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Dosiswerten, die aneinander gereiht angeordnet sind und die Dosisskala ergeben. Vorzugsweise handelt es sich um Zahlenwerte, welche die gewünschte Produktdosis in internationalen Einheiten (IU) angeben.

Alternativ kann die Dosisskala ohne Steigung über den Umfang des Dosisanzeigeelements, wie zum Beispiel des Dosisanzeigerings angeordnet sein, wobei sich die Skalenwerte dann nach einer Umdrehung des Dosisanzeigeelements wiederholen. Bei einer Dosisskala mit Steigerung, das heißt einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte angeordnet werden können. Die Antriebs- und Dosiervorrichtung umfasst ferner eine Zeigeeinrichtung, wobei das Dosisanzeigeelement zur Einstellung der zu verabreichenden Dosis relativ zu der Zeigeeinrichtung und insbesondere um eine Drehachse, die vorzugsweise der Längsachse des Gehäuses oder der Antriebs- und Dosiervorrichtung und/oder des Dosisanzeigeelements entspricht, drehbar ist. Hierbei kann es sich um eine reine Drehbewegung, das heißt eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Produktdosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegung und die Dosisskala vorteilhaft die gleiche Steigung aufweisen können. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann zum Beispiel ein Fenster sein, das von einem Durchbruch im Gehäuse oder einem transparenten Einsatz am oder im Gehäuse gebildet sein kann. Alternativ oder zusätzlich kann die Zeigeeinrichtung ein Zeiger, insbesondere Pfeil, sein oder einen Zeiger aufweisen, der zum Beispiel zusätzlich zu dem Fenster den aktuell eingestellten Dosiswert der Dosisskala markiert, welcher der eingestellten Dosis entspricht. Dies kann zum Beispiel dann von Vorteil sein, wenn in dem Fenster zusätzlich noch mindestens ein anderer Wert zumindest teilweise erscheint, um eine eindeutige Dosisauswahl sicherzustellen. Der Zeiger kann zum Beispiel ein Vorsprung oder ein Aufdruck oder eine Kerbe oder dergleichen am Gehäuse sein.

Die Antriebs- und Dosiervorrichtung umfasst ein Dosierglied, das zum Beispiel als Dosierknopf ausgebildet ist und optional als Dosiseinstellglied bezeichnet werden kann. Das Dosierglied ist vorzugsweise vom Verwender (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vom Verwender ergriffen und relativ zu dem Gehäuse und insbesondere der Zeigeeinrichtung um eine Drehachse, die vorzugsweise der Längsachse der Antriebs- und Dosiervorrichtung entspricht, verdreht. Das Dosierglied ist vorzugsweise axialfest mit dem Gehäuse verbunden, insbesondere entlang einer Längsachse des Gehäuses verschiebefest, wodurch vorteilhaft die intuitive Handhabung der Vorrichtung durch den Verwender erleichtert wird, da er lediglich eine Drehbewegung des Dosierglieds zur Dosiseinstellung auszuführen braucht.

Insbesondere kann das Dosisanzeigeelement zumindest während der Dosiseinstellung verdrehfest, aber zum Beispiel axial verschiebbar mit dem Dosierglied verbunden oder gekoppelt sein. Für die intuitive Bedienung ist es vorteilhaft, wenn das Dosisanzeigeelement bei Drehung des Dosierglieds um einen Drehwinkel ebenfalls um diesen Drehwinkel verdreht wird.

Insbesondere kann zur Einstellung der zu verabreichenden Dosis durch Drehung des Dosierglieds relativ zu der Zeigeeinrichtung das Dosisanzeigeelement aus einer Ausgangsposition, die zum Beispiel eine Nulldosisposition sein kann, in welcher in der Zeigeeinrichtung die Dosis Null ablesbar ist, relativ zu der Zeigeeinrichtung um eine Drehachse drehbar sein. Mittels der Zeigeeinrichtung ist der Dosiswert der Dosisskala ablesbar, welcher der eingestellten Dosis entspricht.

Durch Drehung des Dosierglieds relativ zu der Zeigeeinrichtung in eine erste Drehrichtung kann eine Erhöhung der Dosis bewirkt werden. Durch Drehung des Dosierglieds in eine zweite, der ersten Drehrichtung entgegen gesetzte Drehrichtung kann die eingestellte Dosis verringert oder korrigiert werden.

Insbesondere kann durch Hin- und Herdrehen des Dosierglieds in die erste und zweite Drehrichtung das Dosisanzeigeelement zwischen einer Maximaldosisposition und einer Nulldosisposition (Ausgangsposition) hin und her dreh- oder schraubbar sein. In der Nulldosisposition kann vorteilhaft der Dosiswert oder die Ziffer "0" in der Zeigeeinrichtung ablesbar sein. In der Maximaldosisposition kann vorteilhaft die maximal mit der Antriebs- und Dosiervorrichtung einstellbare Produktdosis ablesbar sein. In der Nulldosisposition kann das Dosisanzeigeelement gegen die Drehung in die zweite Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung bewegt werden, die eine Erhöhung der Dosis bewirkt. In der Maximaldosisposition ist das Dosisanzeigeelement vorzugsweise gegen die Drehung in die erste Drehrichtung, nämlich in die Drehrichtung, welche die Erhöhung der Dosis über die maximal einstellbare Dosis hinausbewirken würde, blockiert. Das Dosisanzeigeelement kann in der Maximaldosisposition, insbesondere nur, in die zweite Drehrichtung gedreht werden, die eine Verringerung der Produktdosis bewirkt.

Das Dosisanzeigeelement kann zum Beispiel einen Anschlag aufweisen, der in der Nulldosisposition an einen Gegenanschlag anschlägt und somit die Drehung in die zweite Drehrichtung verhindert. Der gleiche oder ein zusätzlicher Anschlag des Dosisanzeigeelements kann die Drehung des Dosisanzeigeelements über die Maximaldosisposition hinaus verhindern. Insbesondere kann hierfür ein weiterer Gegenanschlag nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach ein Nulldosisanschlag für den Nulldosisgegenanschlag und/oder einen Maximaldosisanschlag für den Maximaldosisgegenanschlag aufweisen. Vorzugsweise wirken der Anschlag oder die Anschläge in Umfangsrichtung und/oder in Axialrichtung.

Die Antriebs- und Dosiervorrichtung umfasst ferner eine Ausschüttfeder und ein Vortriebsglied, wie zum Beispiel ein Stößel oder eine Kolbenstange, welches auf einen Kolben des Produktbehälters wirkt, insbesondere an dem Kolben anstößt, um den Kolben in den Produktbehälter für die Produktausschüttung zu verschieben.

Die Ausschüttfeder kann die für die Ausschüttung des Produkts erforderliche Energie speichern und für die Produktausschüttung an das Vortriebsglied abgeben, wodurch das Vortriebsglied um einen Gesamtausschütthub, welcher proportional zu der eingestellten Produktdosis ist, in Vortriebsrichtung bewegt werden. Ist zum Beispiel eine Dosis von 30 IU eingestellt, ist der Gesamtausschütthub der Weg, den das Vortriebsglied entlang der Längsachse ausführt, der eine Ausschüttung der 30 IU aus dem Produktbehältern bewirkt.

Durch wiederholtes Dosieren und Ausschütten kann die gesamte in dem Produktbehälter ausschüttbare Produktmenge mit mehreren Gesamtausschütthüben ausgeschüttet werden.

Die Ausschüttfeder kann zum Beispiel so mit dem Dosierglied gekoppelt sein, dass eine Drehung des Dosierglieds in die erste Drehrichtung bei der Dosiseinstellung die Ausschüttfeder vorspannt. Die Feder kann dann die für die Ausschüttung der eingestellten Dosis erforderliche Energie speichern.

Vorzugsweise kann die Feder bei Auslieferung der Antriebs- und Dosiervorrichtung bereits mit so viel Energie vorgespannt sein, dass die Energie für mehrere Ausschüttungen der Produktdosis ausreicht, insbesondere für mehrere Gesamtausschütthübe. Vorteilhaft reicht die in der vorgespannten Feder enthaltene Energie aus, die gesamte aus dem Produktbehälter ausschüttbare Produktmenge mit mehreren Gesamtausschütthüben vollständig auszuschütten. In dieser Alternative kann das Dosierglied bei der Dosiseinstellung von der Feder entkoppelt sein, das heißt nicht so mit der Ausschüttfeder gekoppelt sein, dass eine Drehung des Dosierglieds ein Spannen der Feder bewirkt. Hierdurch lässt sich das Dosierglied für den Verwender bei der Dosiseinstellung mit deutlich weniger Kraftaufwand drehen.

Die Erfindung zeichnet sich dadurch aus, dass das Dosisanzeigeelement erst gegen, d.h. am oder kurz vor dem Ende des Gesamtausschütthubs des Vortriebsglieds relativ zu der Zeigeeinrichtung drehbar ist, insbesondere in seine Ausgangs- oder Nulldosisposition zurückgedreht wird. Hierdurch wird dem Verwender am Ende der Ausschüttung aktiv signalisiert, dass die Ausschüttung beendet oder in wenigen Augenblicken beendet ist.

Der Gesamtausschütthub kann einen ersten Teilausschütthub umfassen, wobei das Dosisanzeigeelement in Bezug auf die Zeigeeinrichtung während des ersten Teilausschütthubs drehfest ist und nach dem ersten Teilausschütthub relativ zu der Zeigeeinrichtung drehbar ist. Insbesondere kann die Antriebs- und Dosiervorrichtung eine Feder, insbesondere eine Anzeigerückstellfeder umfassen, welche das Dosisanzeigeelement in seine Ausgangs- oder Nulldosisposition zurückdreht. Zum Beispiel kann diese Feder als Druckfeder oder Drehfeder wirkend sein. Die Feder kann durch Drehen des Dosierglieds in die erste Drehrichtung gespannt und durch Drehung des Dosierglieds in die zweite Drehrichtung entspannt werden. Während des ersten Teilausschütthubs kann sich die Feder nicht entspannen, bleibt also gespannt, wobei das Dosisanzeigeelement in Bezug auf die Zeigeeinrichtung oder das Gehäuse rotatorisch stillsteht und das Dosisanzeigeelement, wenn der erste Teilausschütthub erfolgt ist, sich insbesondere während eines zweiten Teilausschütthubs des Vortriebsglieds in die Nulldosis- oder Ausgangsposition zurückdreht.

Insbesondere kann der Gesamtausschütthub den ersten Teilausschütthub und einen zweiten Teilausschütthub umfassen oder daraus bestehen. Das Dosisanzeigeelement ist während des zweiten Teilausschütthubs relativ zu der Zeigeeinrichtung oder dem Gehäuse drehbar, insbesondere schraubbar. Vorzugsweise ist der zweite Teilausschütthub kleiner als der erste Teilausschütthub. Der zweite Teilausschütthub ist vorzugsweise betreffend seine Länge fest oder konstruktiv vorgegeben, wobei der erste Teilausschütthub variabel und/oder abhängig von der eingestellten Produktdosis ist.

Der zweite Teilausschütthub kann zum Beispiel kleiner gleich 5 IU oder vorzugsweise kleiner gleich 2 oder kleiner gleich 1 IU sein. Dadurch, dass der zweite Teilausschütthub relativ klein ist, insbesondere nur 1, 2 oder wenige IU groß ist, wird erreicht, dass sich das Dosisanzeigeelement erst gegen Ende des Gesamtausschütthubs in seine Ausgangs- oder Nulldosisposition zurückdreht oder schraubt. Insbesondere ist das Ende des zweiten Teilausschütthubs auch das Ende des Gesamtausschütthubs. Das Ende des ersten Teilausschütthubs ist vorzugsweise der Anfang des zweiten Teilausschütthubs.

Die Antriebs- und Dosiervorrichtung kann in bevorzugten Ausführungen eine Kupplung, insbesondere eine Anzeigerückstellkupplung aufweisen, welche die Zeigeeinrichtung oder/und das Gehäuse während des ersten Teilausschütthubs drehfest mit dem Dosisanzeigeelement koppelt und während des zweiten Teilausschütthubs rotatorisch entkoppelt. Die geschlossene Kupplung koppelt die Zeigeeinrichtung oder das Gehäuse drehfest mit dem Dosisanzeigeelement, wobei die geöffnete Kupplung die Zeigeeinrichtung und/oder das Gehäuse von dem Dosisanzeigeelement rotatorisch entkoppelt. Die geöffnete Kupplung erlaubt, dass das Dosisanzeigeelement relativ zu der Zeigeeinrichtung oder dem Gehäuse drehbar ist, insbesondere mittels der Anzeigerückstellfeder in Richtung seiner Ausgangs- oder Nulldosisposition gedreht wird.

Vorzugsweise weist die Kupplung eine erste Kupplungsstruktur auf, welche von einer im Bezug auf das Dosierglied drehfesten und axial verschiebbaren Dosierhülse gebildet wird, und eine zweite Kupplungsstruktur auf, welche von einem drehfest und axial verschiebbaren Rotationsglied gebildet wird, welches drehfest und axial verschiebbar mit dem Dosisanzeigeelement verbunden ist. Die erste und zweite Kupplungsstruktur sind während des ersten Teilausschütthubs in einem verdrehgesicherten Eingriff, wobei dieser Eingriff während des zweiten Teilausschütthubs gelöst ist.

Vorzugsweise ist die erste Kupplungsstruktur, insbesondere die Dosierhülse so mit dem Vortriebsglied gekoppelt oder in einem Eingriff, dass die erste Kupplungsstruktur, insbesondere die Dosierhülse zusammen mit dem Vortriebsglied gemeinsam um den ersten Teilausschütthub und vorzugsweise auch um den zweiten Teilausschütthub bewegt werden.

Insbesondere kann die Kupplung geschlossen werden, oder die erste Kupplungsstruktur in den verdrehgesicherten Eingriff mit der zweiten Kupplungsstruktur bewegt werden, wenn das Dosisanzeigeelement aus seiner Ausgangs- oder Nulldosisposition mittels Drehung des Dosierglieds in die erste Drehrichtung bewegt wird.

Insbesondere wird die Kupplung geöffnet, wenn das Dosisanzeigeelement mittels Drehung des Dosierglieds in die zweite Drehrichtung in seine Ausgangs- oder Nulldosisposition bewegt wird.

Vorzugsweise weist die Dosierhülse ein Innengewinde auf, welches in ein Außengewinde des Vortriebsglieds eingreift und durch Drehung des Dosierglieds relativ zu dem Vortriebsglied schraubbar ist. Mittels Drehung des Dosierglieds in die erste Drehrichtung, welche eine Dosiserhöhung bewirkt, kann ein Dosierabstand zwischen dem Dosierglied und einem Dosieranschlag erhöht werden. Insbesondere kann der Dosierabstand zwischen dem Dosieranschlag und der Dosierhülse durch Drehung des Dosierglieds in die zweite Drehrichtung, welche eine Dosisverringerung bewirkt, verringert werden. Insbesondere entspricht der Abstand zwischen der Dosierhülse und dem Dosieranschlag dem Gesamtausschütthub des Vortriebsglieds.

Der Dosieranschlag ist vorzugsweise ein in Axialrichtung wirkender Anschlag und kann zum Beispiel von dem Gehäuse oder einem gehäusefesten Element, wie zum Beispiel einem Gehäuseeinsatz, der dreh- und axialfest mit dem Gehäuse verbunden ist und daher dem Gehäuse zugerechnet werden kann, gebildet werden.

Die erste Kupplungsstruktur und die zweite Kupplungsstruktur können jeweils eine über den Umlauf verlaufende Verzahnung sein, wobei die erste Kupplungsstruktur vorzugsweise eine Außenverzahnung ist und die zweite Kupplungsstruktur vorzugsweise eine Innenverzahnung ist, oder umgekehrt.

Vorzugsweise ist die erste Kupplungsstruktur bei geschlossener Kupplung relativ zu der zweiten Kupplungsstruktur entlang der Längsachse der Antriebs- und Dosiervorrichtung bewegbar.

Das Rotationsglied ist vorzugsweise in Bezug auf das Dosierglied und/oder das Gehäuse und/oder die Zeigeeinrichtung drehbar und axialfest. Insbesondere ist das Rotationsglied in einem drehbaren und axialfesten Eingriff mit dem Dosierglied oder dem Gehäuse.

Die Ausschüttfeder kann eine als Druckfeder wirkende Feder, insbesondere Wendelfeder sein. Alternativ kann die Ausschüttfeder eine als Drehfeder wirkende, wie zum Beispiel wendel- oder spiralförmige Feder sein.

Die vorzugsweise als Druckfeder wirkende Ausschüttfeder kann zum Beispiel auf Druck vorgespannt sein und auf das Vortriebsglied wirken, und sich insbesondere an dem Vortriebsglied und dem Gehäuse oder einem zumindest axialfest mit dem Gehäuse verbundenen Element, wie zum Beispiel dem Dosierglied abstützen. Zum Beispiel kann das Vortriebsglied hülsenförmig sein, wobei die Ausschüttfeder zumindest teilweise innerhalb des hülsenförmigen Vortriebsglieds angeordnet sein kann. Die Ausschüttfeder kann sich mit ihrem distalen Ende an dem Vortriebsglied, insbesondere an einer nach innen ragenden Schulter z.B. am distalen Ende des Vortriebsglieds, und mit ihrem proximalen Ende an dem Gehäuse oder dem zumindest axialfest mit dem Gehäuse verbundenen Element abstützen.

Das Vortriebsglied ist vorzugsweise relativ zu dem Gehäuse oder der Zeigeeinrichtung drehfest und axial entlang der Längsachse bewegbar, insbesondere in einem verdrehgesicherten und axial bewegbaren Eingriff mit dem Gehäuse oder einem gehäusefesten Element. Zum Beispiel kann das Vortriebsglied eine Längsführung, wie zum Beispiel eine Längsnut aufweisen, in welche das Gehäuse oder ein gehäusefestes Element eingreift und das Vortriebsglied verdrehgesichert und entlang der Längsachse verschiebbar führt.

Die Antriebs- und Dosiervorrichtung kann ferner eine Greifeinrichtung umfassen, welche das Vortriebsglied axial festhält, wobei der Eingriff der Greifeinrichtung lösbar ist, so dass das Vortriebsglied relativ zu der Greifeinrichtung entlang der Längsachse der Antriebs- und Dosiervorrichtung bewegbar ist. Die Greifeinrichtung kann zum Beispiel in ein Außengewinde oder eine Verzahnung des insbesondere in Bezug auf das Gehäuse verdrehgesicherten aber axial verschiebbaren Vortriebsglieds eingreifen, wodurch die Greifeinrichtung das Vortriebsglied gegen eine Verschiebung entlang der Längsachse insbesondere in distale Richtung sichert oder blockiert. Der Eingriff der Greifeinrichtung in das Vortriebsglied kann zum Beispiel durch Betätigung eines Betätigungsglieds lösbar sein, wodurch das Vortriebsglied insbesondere mittels der vorgespannten Ausschüttfeder in distale Richtung oder Ausschüttrichtung verschiebbar ist.

Beispielsweise kann die Greifeinrichtung ein insbesondere federnd angeordnetes Halteeingriffsglied aufweisen, welches in das Vortriebsglied, insbesondere in dessen Außengewinde oder Verzahnung, eingreift. Das federnd angeordnete Halteingriffsglied kann so vorgespannt sein, dass es aufgrund der Vorspannung aus dem Eingriff mit dem Vortriebsglied bewegt wird. Das Halteeingriffsglied kann zum Beispiel von dem Gehäuse oder einem gehäusefesten Element oder einem Greifring gebildet werden, wobei das Gehäuse oder das gehäusefeste Element oder der Greifring die federnde Anordnung des Halteeingriffsglieds bewirkt.

Die Greifeinrichtung kann ferner ein Klemmstück, insbesondere eine Klemmhülse aufweisen, welche relativ zu dem Halteeingriffsglied entlang der Längsachse zwischen einer Klemmposition und einer Freigabeposition hin und her bewegbar ist, wobei das Klemmstück das Halteeingriffsglied in den Eingriff mit dem Vortriebsglied drückt, wenn es in seiner Klemmposition ist und in seiner Freigabeposition das Halteeingriffsglied freigibt, so dass das Halteeingriffglied aus dem die Axialbewegung sichernden Eingriff mit dem Vortriebsglied bewegbar ist. Die Greifeinrichtung kann zum Beispiel eine auf das Klemmstück wirkende Feder, insbesondere eine als Druckfeder wirkende Feder aufweisen, welche das Klemmstück in seiner Klemmposition hält. Durch Betätigung des Betätigungsglieds kann das Klemmstück unter Vorspannen der Feder, die auch als Klemmstückrücksetzfeder bezeichnet werden kann, verschoben werden, wodurch das Halteeingriffsglied freigegeben wird. Das Klemmstück weist vorzugsweise eine gegenüber der Längsachse geneigte, insbesondere in einem spitzen Winkel oder konisch geneigte Fläche auf, welche an dem Halteeingriffsglied entlang gleitet. Wird das Betätigungsglied nicht mehr betätigt, drückt die Klemmstückrücksetzfeder das Klemmstück in seine Halteposition zurück.

Die Antriebs- und Dosiervorrichtung umfasst in bevorzugten Ausführungsformen ein Lagerelement, mit dem das Dosisanzeigeelement in einem Eingriff ist. Dieser Eingriff bewirkt vorteilhaft die Dreh- und/oder Schraubbewegung des Dosisanzeigeelements relativ zu der Zeigeeinrichtung. Zum Beispiel kann der Eingriff zwischen Dosisanzeigeelement und Lagerelement ein Gewindeeingriff sein. Insbesondere kann das Lagerelement ein Außengewinde und das Dosisanzeigeelement ein Innengewinde aufweisen, wobei diese Gewinde ineinander greifen und dadurch bewirken, dass das Dosisanzeigeelement relativ zu dem Lagerelement schraubbar ist. Alternativ kann das Dosisanzeigeelement in einem axialfesten und drehbaren Eingriff mit dem Lagerelement sein, was insbesondere bei einem Dosisanzeigering vorteilhaft ist.

Das Lagerelement ist in Bezug auf das Gehäuse bevorzugt drehfest und axial verschiebbar. Insbesondere kann das Lagerelement in das Gehäuse drehfest und axial verschiebbar eingreifen. Das Gehäuse kann zum Beispiel eine Längsnut aufweisen, in welche das Lagerelement eingreift. Das Lagerelement ist vorzugsweise in distale Richtung gegen die Kraft einer insbesondere als Druckfeder wirkenden Rücksetzfeder verschiebbar, insbesondere durch Betätigung eines Betätigungsglieds. Die Rücksetzfeder kann das Lagerelement in proximale Richtung zurücksetzen, insbesondere unter Zurücksetzung des Betätigungsglieds. Die Rücksetzfeder für das Lagerelement kann zusätzlich die Aufgabe der Klemmstückrücksetzfeder übernehmen, so dass die Rücksetzfeder mindestens eine Doppelfunktion hat.

Das Lagerelement kann zum Beispiel axialfest mit dem Klemmstück verbunden sein oder das Klemmstück bilden. Hierdurch wird bewirkt, dass das Klemmstück zusammen mit dem Lagerelement entlang der Längsachse verschoben werden kann. Insbesondere kann das Dosisanzeigeelement zusammen mit dem Lagerelement zusätzlich zu einer eventuellen Schraubbewegung verschoben werden.

Die Antriebs- und Dosiervorrichtung kann z.B. ein Kupplungsglied aufweisen, welches mittels einer Kupplung, insbesondere einer lösbaren Dosierkupplung verdrehfest mit dem Dosierglied verbunden ist. Das Dosierglied ist für die Dosiseinstellung mit dem Kupplungsglied verdrehgesichert verbunden, d.h. die Kupplung ist geschlossen, wobei die Kupplung für die Ausschüttung der eingestellten Dosis geöffnet ist, so dass das Kupplungsglied relativ zu dem Dosierglied drehbar ist. Das Kupplungsglied kann eine dritte Kupplungsstruktur, wie z.B. eine über den Umfang umlaufende Verzahnung, insbesondere Außenverzahnung, aufweisen. Das Dosierglied kann eine vierte Kupplungsstruktur, wie z.B. eine über den Umfang umlaufende Verzahnung, insbesondere Innenverzahnung aufweisen. Ist die Kupplung geschlossen ist die dritte Kupplungsstruktur in einem verdrehgesicherten Eingriff mit der vierten Kupplungsstruktur, wobei die dritte Kupplungsstruktur aus dem Eingriff mit der vierten Kupplungsstruktur ist, wenn die Kupplung geöffnet ist. Die Kupplung wird vorzugsweise von einer Kupplungsfeder in eine geschlossene Position gedrückt. Die Kupplungsfeder kann z.B. eine als Druckfeder wirkende Wendelfeder sein. Die Aufgabe der Kupplungsfeder kann z.B. von der Rücksetzfeder übernommen werden, so dass die Rücksetzfeder das Kupplungsglied in dem verdrehgesicherten Eingriff mit dem Dosierglied hält. Die Kupplung kann vorzugsweise durch Betätigung eines Betätigungsglieds geöffnet werden, insbesondere gegen die Kraft der Kupplungsfeder. Das Kupplungsglied kann z.B. so an das Lagerelement angreifen, dass es das Lagerelement insbesondere mittels Betätigung des Betätigungsglieds in distale Richtung verschiebt.

Das Kupplungsglied kann vorzugsweise permanent verdrehfest und axial verschiebbar mit dem Dosisanzeigeelement und/oder dem Rotationsglied verbunden sein. Über die zwischen Dosierglied und Kupplungsglied gebildete, geschlossene Kupplung kann das Dosisanzeigeelement und/oder das Rotationsglied drehfest mit dem Dosierglied gekoppelt werden, wobei die drehfeste Kopplung durch Öffnen der Kupplung gelöst werden kann.

Die Antriebs- und Dosiervorrichtung kann zum Beispiel ein vom Verwender der Antriebs- und Dosiervorrichtung für die Produktausschüttung betätigbares, insbesondere drückbares Betätigungsglied umfassen. Das Betätigungsglied kann in der Gestalt eines Betätigungsknopfs gebildet sein. Das Betätigungsglied kann eine äußere Oberfläche der Antriebs- und Dosiervorrichtung bilden und/oder von außen zugänglich sein. Das Betätigungsglied kann am proximalen, insbesondere hinteren Ende der Antriebs- und Dosiervorrichtung gebildet sein oder dessen Ende bilden. Das Betätigungsglied lässt sich auf diese Weise vorteilhaft mit dem Daumen der Hand, die das Gehäuse umgreift, betätigen, insbesondere drücken. Durch Loslassen des Betätigungsglieds kann die Betätigung beendet werden. Unter "betätigen" wird die Verschiebung des Betätigungsglieds in die Antriebs- und Dosiervorrichtung, insbesondere in distale Richtung verstanden, wodurch insbesondere eine Produktausschüttung bewirkt werden kann. Das Betätigungsglied ist vorteilhaft relativ zu dem Dosierglied verschiebbar und kann insbesondere von dem Dosierglied axial verschiebbar aufgenommen sein. Vorzugsweise wird das Dosisanzeigeelement während der Dosiseinstellung relativ zu dem Betätigungsglied zumindest axial bewegt oder sogar geschraubt. Insbesondere ist das Dosisanzeigeelement mittels Drehung des Dosierglieds in die erste und/oder zweite Drehrichtung relativ zu dem Betätigungsglied axial bewegbar. Vorzugsweise ist das Betätigungsglied so mit der Greifeinrichtung, insbesondere dem Klemmstück gekoppelt, dass eine Betätigung des Betätigungsglieds bewirkt, dass der Eingriff der Betätigungseinrichtung, insbesondere des Halteeingriffsglieds in das Vortriebsglied gelöst wird, wodurch das Vortriebsglied in Ausschüttrichtung bewegt wird. Insbesondere bewirkt die Betätigung des Betätigungsglieds eine Verschiebung des Klemmstücks in seine Freigabeposition.

Alternativ oder zusätzlich bewirkt die Betätigung des Betätigungsglieds, dass das Lagerelement zusammen mit dem Dosisanzeigeelement relativ zu der Zeigeeinrichtung entlang der Längsachse verschoben wird. Hierdurch kann an der Zeigeeinrichtung und/oder an dem Dosisanzeigeelement abgelesen werden, dass das Lagerelement zusammen mit dem Dosisanzeigeelement verschoben wurde. Hierdurch lässt sich durch den Verwender kontrollieren, in welchem Betriebszustand sich die Antriebs- und Dosiervorrichtung befindet, das heißt ob die Antriebs- und Dosiervorrichtung insbesondere das Betätigungsglied für eine Ausschüttung betätigt oder unbetätigt ist. In einer Variante kann das Dosisanzeigeelement zusätzlich zu der Dosisskala eine Markierung aufweisen, welche nur dann sichtbar ist, wenn das Betätigungsglied betätigt ist oder das Lagerelement in Bezug auf die Zeigeeinrichtung verschoben ist.

Das Betätigungsglied ist vorzugsweise gegen die Kraft einer Rücksetzfeder, insbesondere der Rücksetzfeder, welche das Lagerelement und/oder das Klemmstück und/der das Kupplungsglied zurücksetzt, betätigbar, wobei die Rücksetzfeder das unbetätigte Betätigungsglied wieder in die unbetätigte Position zurücksetzen kann.

Alternativ oder zusätzlich bewirkt die Betätigung des Betätigungsglieds mittelbar, dass die Dosierhülse zu dem Dosieranschlag hin bewegt wird, nämlich durch Freigabe des Vortriebsglieds, so dass die Antriebsfeder das Vortriebsglied zusammen mit der Dosierhülse zu dem Dosieranschlag hin bewegt. Mit anderen Worten, die Betätigung des Betätigungsglieds gibt das Vortriebsglied für seine Bewegung in Vortriebsrichtung oder in distale Richtung frei.

In besonders bevorzugten Ausführungen verbleibt das Betätigungsglied selbst tätig so lange in seiner gedrückten oder betätigten Position, bis die Produktausschüttung beendet ist oder bis das Dosisanzeigeelement zumindest teilweise, insbesondere zum größten Teil oder vollständig in seine Ausgangs- oder Nulldosisposition zurückgedreht ist. Dies bedeutet, dass das Betätigungsglied in seiner betätigten Position verbleibt, auch wenn der Verwender das Betätigungsglied nicht mehr drückt. Hierdurch wird vorteilhaft sichergestellt, dass - sofern die Produktausschüttung einmal ausgelöst ist - die eingestellte Produktdosis vollständig verabreicht wird.

Insbesondere kann das Betätigungsglied bei seiner Betätigung mit dem Dosierglied verrasten, wobei die Rastverbindung mittels des insbesondere sich zurückdrehenden Dosisanzeigeelements lösbar ist. Hierfür kann das Betätigungsglied wenigstens ein Rastglied aufweisen, welches in das Dosierglied einrastet und von dem Dosisanzeigeelement aus der Rastverbindung gelöst wird.

Die Erfindung wurde anhand mehrerer bevorzugter Ausführungen beschrieben. Weitere bevorzugte Ausführungen werden im Folgenden anhand von Figuren beschrieben. Die dabei offenbarten Merkmale bilden den Gegenstand optional auch mit den vorhergehenden Merkmalen je einzeln und in jeglicher Merkmalskombination vorteilhaft weiter. Es zeigen:
- Figur 1: eine Explosionsdarstellung der Einzelteile einer Injektionsvorrichtung mit einer Antriebs- und Dosiervorrichtung nach einer ersten Ausführungsform,
- Figur 2: die Darstellung aus Figur 1, wobei die Einzelteile im Schnitt dargestellt sind,
- Figuren 3a bis 3d: verschiedene Ansichten einer aus den Einzelteilen aus den Figuren 1 und 2 zusammengesetzten Injektionsvorrichtung in einem Ausgangs- oder Auslieferungszustand,
- Figuren 4a bis 4d: die Ansichten der Vorrichtung aus den Figuren 3a bis 3d mit einer maximal eingestellten Dosis,
- Figuren 5a bis 5d: die Ansichten der Vorrichtung aus den Figuren 3a bis 3d nach Ausschüttung der eingestellten Produktdosis,
- Figuren 6a bis 6d: die Ansichten der Vorrichtung aus den Figuren 3a bis 3d in einem Zustand, bei dem die in dem Produktbehälter enthaltene ausschüttbare Produktdosis geringer als die mit der Vorrichtung maximal einstellbare Dosis ist,
- Figur 7: eine Explosionsdarstellung der Einzelteile einer Injektionsvorrichtung mit einer Antriebs- und Dosiervorrichtung nach einer zweiten Ausführungsform,
- Figur 8: die Darstellung aus Figur 7, wobei die Einzelteile im Schnitt dargestellt sind,
- Figuren 9a bis 9d: verschiedene Ansichten einer aus den Einzelteilen aus den Figuren 7 und 8 zusammengesetzten Injektionsvorrichtung in einem Ausgangs- oder Auslieferungszustand,
- Figuren 10a bis 10d: die Ansichten der Vorrichtung aus den Figuren 9a bis 9d mit einer maximal eingestellten Dosis,
- Figuren 11a bis 11d: die Ansichten der Vorrichtung aus den Figuren 9a bis 9d nach Ausschüttung der eingestellten Produktdosis,
- Figuren 12a bis 12d: die Ansichten der Vorrichtung aus den Figuren 9a bis 9d in einem Zustand, bei dem die in dem Produktbehälter enthaltene ausschüttbare Produktdosis geringer als die mit der Vorrichtung maximal einstellbare Dosis ist,
- Figur 13: eine Explosionsdarstellung der Einzelteile einer Injektionsvorrichtung mit einer Antriebs- und Dosiervorrichtung nach einer dritten Ausführungsform,
- Figur 14: die Darstellung aus Figur 13, wobei die Einzelteile im Schnitt dargestellt sind,

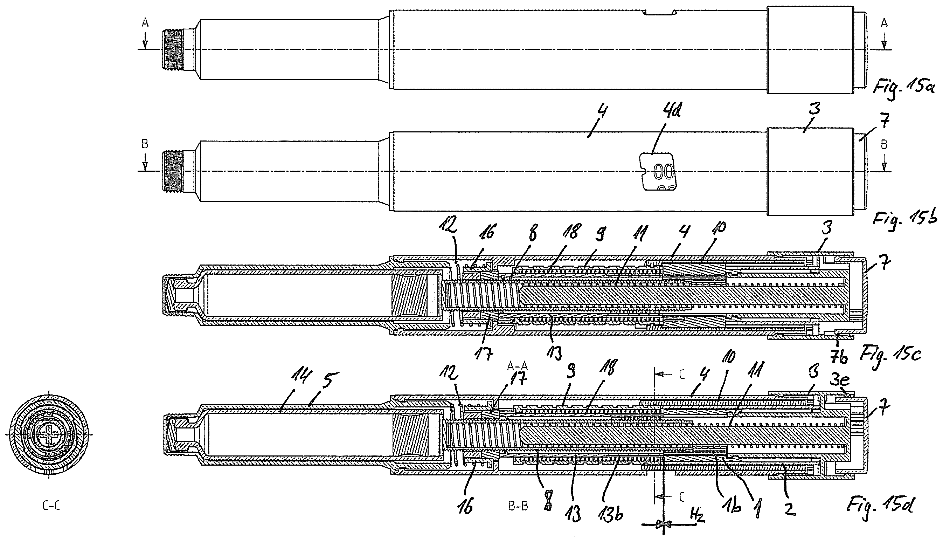

- Figuren 15a bis 15d: verschiedene Ansichten einer aus den Einzelteilen aus den Figuren 13 und 14 zusammengesetzten Injektionsvorrichtung in einem Ausgangs- oder Auslieferungszustand,
- Figuren 16a bis 16d: die Ansichten der Vorrichtung aus den Figuren 15a bis 15d mit einer maximal eingestellten Dosis,

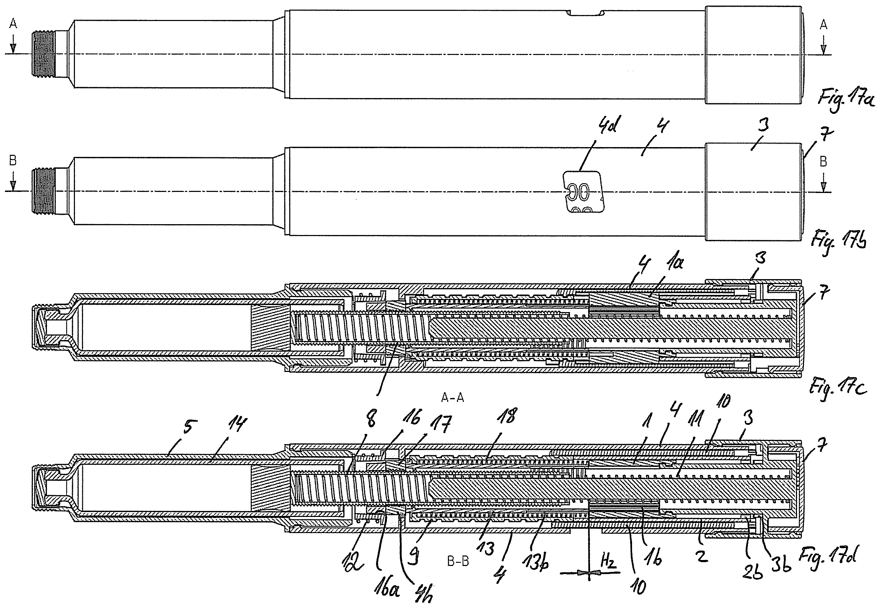

- Figuren 17a bis 17d: die Ansichten der Vorrichtung aus den Figuren 15a bis 15d nach Ausschüttung der eingestellten Produktdosis und

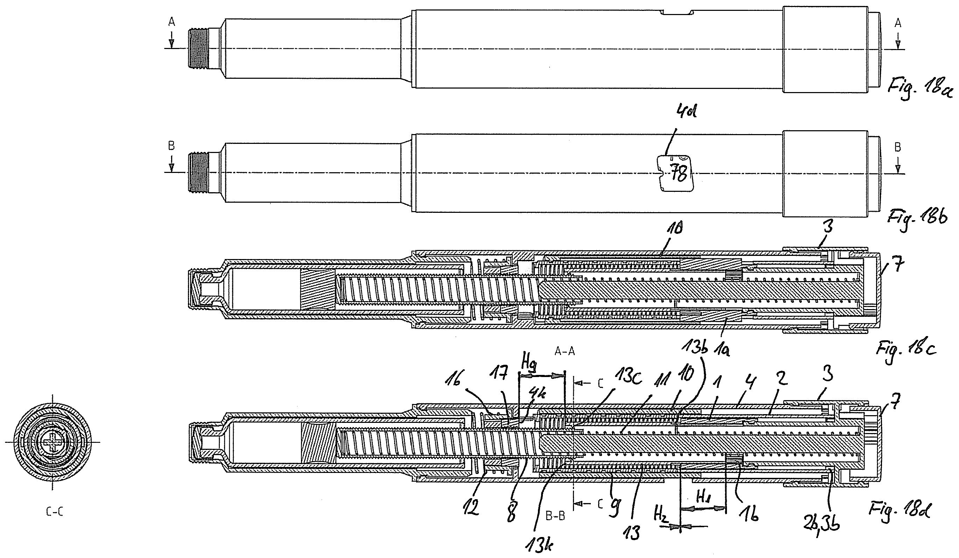

- Figuren 18a bis 18d: die Ansichten der Vorrichtung aus den Figuren 15a bis 15d in einem Zustand, bei dem die in dem Produktbehälter enthaltene ausschüttbare Produktdosis geringer als die mit der Vorrichtung maximal einstellbare Dosis ist.

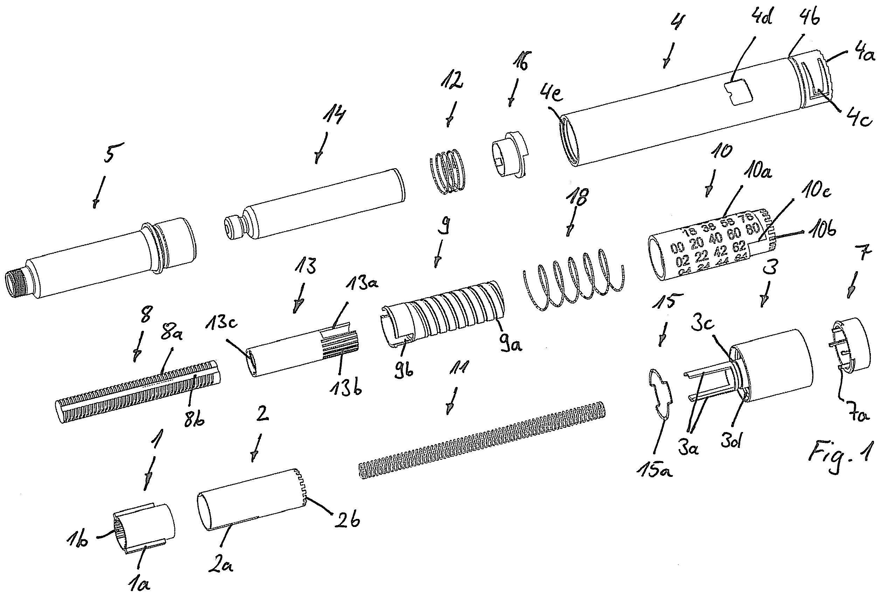

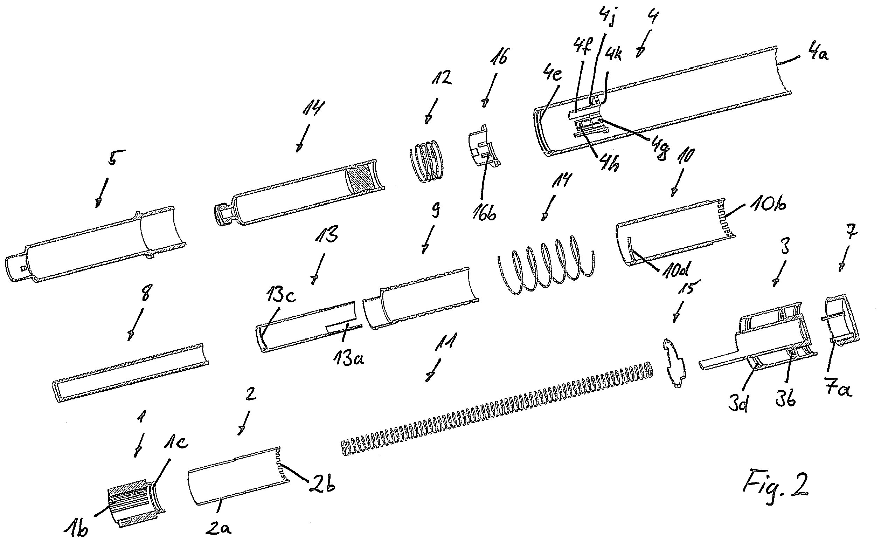

Zunächst wird die erste Ausführungsform anhand der Figuren 1 bis 6d beschrieben. Die zweiten und dritten Ausführungsformen sind der ersten Ausführungsform ähnlich. Um Wiederholungen zu vermeiden, werden für die zweite und dritte Ausführungsform lediglich die Merkmale mit denen sie sich von der ersten Ausführungsform unterscheiden, beschrieben. Gleiche Bezugszeichen bezeichnen funktionell und/oder strukturell gleichartige Bauteile.

Die Antriebs- und Dosiervorrichtung weist ein hülsenförmiges Gehäuse 4 auf, in dem ein fensterartiger Durchbruch zur Bildung einer Zeigeeinrichtung 4d angeordnet ist. An dem distalen, das heißt vorderen Ende des Gehäuses 4 ist ein Produktbehälterhalter 5 formschlüssig, vorzugsweise unlösbar befestigt, insbesondere verschnappt, welcher einen Produktbehälter 14 in der Gestalt einer Karpule aufnimmt. Die Karpule weist ein zylindrisches Gehäuse auf, in dem ein Kolben verschiebbar angeordnet ist. Am distalen Ende weist die Karpule ein mit einer Nadel durchstechbares Septum auf. Zwischen dem Septum und dem Kolben befindet sich das zu verabreichende Produkt. Durch Verschiebung des Kolbens Richtung Septum wird das Produkt aus dem Produktbehälter 14 verdrängt. An dem proximalen Ende des Produktbehälters ist ein Gewinde oder ein Bajonettverschluss gebildet, an dem die Nadel befestigbar ist. Auf dem Produktbehälterhalter kann eine Kappe (nicht gezeigt) abnehmbar aufgesteckt werden. An dem proximalen, das heißt hinteren Ende des Gehäuses 4 ist ein relativ zu dem Gehäuse 4 drehbares Dosierglied 3 angeordnet, welches eine äußere Oberfläche der Vorrichtung bildet und vom Verwender der Vorrichtung greifbar ist und relativ zu dem Gehäuse 4 verdrehbar ist. Eine Drehung des Dosierglieds 3 in eine erste Drehrichtung bewirkt eine Erhöhung der Dosis, wobei die Drehung des Dosierglieds in eine zweite Drehrichtung eine Verringerung der Dosis bewirkt. Das Dosierglied 3 ist axialfest mit dem Gehäuse 4 verbunden. Das Gehäuse 4 weist eine Ringnut 4b auf, in die ein Ringsteg 3d am inneren Umfang des Dosierglieds einrastet, wodurch das Dosierglied drehbar und axialfest mit dem Gehäuse 4 verbunden ist.

Insbesondere im Bereich des proximalen Endes des Gehäuses 4 ist ein in Umfangsrichtung wirkender Nulldosisgegenanschlag 4c für einen Nulldosisanschlag 10c eines Dosisanzeigeelements 10 gebildet. Der Nulldosisgegenanschlag 4c ist - insbesondere aufgrund der einfacheren Montage - federnd an einem Arm angeordnet.

Am proximalen Ende der Antriebs- und Dosiervorrichtung ist ein als Betätigungsknopf ausgestattetes Betätigungsglied 7 angeordnet, welches durch den Verwender der Vorrichtung für eine Produktausschüttung betätigbar, insbesondere in distale Richtung drückbar ist. Das Betätigungsglied 7 ist in Bezug auf das Dosierglied 3 so angeordnet dass es seine Axialposition während der Dosiseinstellung hierzu nicht ändert. Das Betätigungsglied 7 ist insbesondere um einen Betätigungshub verschiebbar in dem Dosierglied 3 aufgenommen. Das Betätigungsglied 7 ist gegen die Kraft einer vorgespannten Rücksetzfeder 12 betätigbar, welche als Wendelfeder ausgestaltet ist und als Druckfeder wirkt. Die Rücksetzfeder 12 stützt sich an den Produktbehälterhalter 5, alternativ an den Produktbehälter 14, und an einem hülsenförmigen Klemmstück 16, das im Folgenden als Klemmhülse 16 bezeichnet wird, ab. Die Klemmhülse 16 wird durch Betätigung des Betätigungsglieds 7 aus einer Klemm- oder Halteposition in eine Freigabeposition verschoben. Die Feder 12 setzt die Klemmhülse 16 aus der Freigabeposition in die Klemm- oder Halteposition zurück, insbesondere auch das Betätigungsglied 7.

Die Klemmhülse 16 ist axialfest mit einem hülsenförmigen Lagerelement 9 verbunden, welches ein Außengewinde 9a aufweist und mittels einer Ausnehmung 9b verdrehfest und axial verschiebbar in das Gehäuse 4 eingreift. Das Lagerelement 9 ist somit zusammen mit der Klemmhülse 16 entlang der Längsachse L verschiebbar. Zwischen dem Betätigungsglied 7 und dem Lagerelement 9 ist ein Kupplungsglied 2 angeordnet, welche mit einer nutförmigen Ausnehmung 2a drehfest und axial verschiebbar mit einem Steg 1a eines hülsenförmigen Rotationsglieds 1, insbesondere permanent, drehfest verbunden ist, so dass das Kupplungsglied 2 eine Drehung des Rotationsglieds 1 mitmacht. Das Kupplungsglied 2 stößt mit seinem distalen Ende lose an das proximale Ende des Lagerelements 9 an. Das Kupplungsglied 2 weist an seinem proximalen Ende eine über den Umfang sich erstreckende dritte Kupplungsstruktur 2b in der Gestalt einer Verzahnung auf. Die dritte Kupplungsstruktur 2b ist Teil einer Kupplung 2b, 3b, welche das Dosierglied 3 verdrehgesichert mit dem Kupplungsglied 2 verbindet, wenn die Kupplung 2b, 3b geschlossen, d.h. die dritte Kupplungsstruktur 2b in einem Eingriff mit einer vierten Kupplungsstruktur 3b, welche von dem Dosierglied 3 gebildet wird, ist. Wenn die Kupplung 2b, 3b geöffnet ist, ist das Kupplungsglied 2 relativ zu dem Dosierglied 3 drehbar.

Das Betätigungsglied 7, insbesondere dessen mindestens eine Rastglied 7a stößt an das proximale Ende des hülsenförmigen Kupplungsglieds 2 an, wodurch eine Betätigung des Betätigungsglieds 7 eine Verschiebung der Kupplungshülse 2 in distale Richtung und somit auch eine Verschiebung des hülsenförmigen Lagerelements 9 und der Klemmhülse 16 in distale Richtung bewirkt.

Das als Dosisanzeigehülse ausgestattete Dosisanzeigeelement 10 weist über seinen Umfang eine mehrfach umlaufende, wendelförmige oder helixförmige Dosisskala 10a auf, welche aus einer Vielzahl aneinander gereihten, insbesondere in internationalen Einheiten (IU) angegebenen Dosiswerten gebildet wird. Die Dosisskala 10a kann zum Beispiel in Einser- oder Zweierschritten die einstellbaren Dosiswerte von 0 bis 60 oder 80 IU aufweisen. Durch Drehung des Dosierglieds 3 relativ zu dem Gehäuse 4 oder der Zeigeeinrichtung 4d kann die gewünschte auszuschüttende Produktdosis eingestellt werden, wobei der entsprechende Dosiswert an der Zeigeeinrichtung 4 d ablesbar ist oder in der Zeigeeinrichtung 4d erscheint.

Das Dosisanzeigeelement 10 ist insbesondere permanent drehfest und axial verschiebbar in Bezug auf das Kupplungsglied 2, wobei der Steg 1a des Rotationsglieds 1 durch die nutförmige Ausnehmung 2a des Kupplungsglieds 2 hindurch greift und drehfest und axial verschiebbar in eine Längsführung (nicht gezeigt) des Dosisanzeigeelements 10 eingreift.

Das Dosisanzeigeelement 10 weist ein Innengewinde 10d auf, welches in das Außengewinde 9a des Lagerelements 9 eingreift, so dass das Dosisanzeigeelement 10 an dem Lagerelement 9 entlang schraubbar ist.

Das Dosisanzeigeelement 10 weist an seinem proximalen Ende eine Verzahnung 10b auf, welche dazu dient, das mindestens eine mit dem Dosierglied 3 verrastete Rastglied 7a des Betätigungsglieds 7 aus dem Rasteingriff zu lösen, so dass das Betätigungsglied 7 in proximale Richtung zurückgesetzt werden kann, insbesondere mittels der Rücksetzfeder 12.

Die dritte Kupplungsstruktur 2b des Kupplungsglieds 2 ist in einem verdrehgesicherten Eingriff mit der vierten Kupplungsstruktur 3b des Dosierglieds 3, so dass das Kupplungsglied 2 mit dem Dosierglied 3 während der Dosiseinstellung mitgedreht wird, wobei das Dosierglied 2 das Rotationsglied 1 und das Dosisanzeigeelement 10 ebenfalls mitdreht. Hierdurch wird das Dosisanzeigeelement 10 an dem Lagerelement 9 entlang geschraubt, wodurch die eingestellte Produktdosis in der Zeigeeinrichtung 4d ablesbar ist.

Das Rotationsglied 1 ist z.B. mittels seines in eine Ringnut 3c des Dosierglieds 3 eingreifenden Ringstegs 1c relativ zu dem Dosierglied 3 axialfest und drehbar verbunden. Die Drehbarkeit des Rotationsglieds 1 relativ zu dem Dosierglied 3 ist abhängig von dem Schaltzustand der Kupplung 2b, 3b, wobei das Rotationsglied 1 relativ zu dem Dosierglied 3 drehbar ist, wenn die Kupplung 2b, 3b geöffnet, das heißt das Betätigungsglied 7 betätigt ist und drehfest ist, wenn die Kupplung 2b, 3b geschlossen ist, das heißt das Betätigungsglied 7 unbetätigt ist.

Das Dosierglied 3 weist mindestens einen, hier zwei Stege 3a auf, welche insbesondere permanent in einem verdrehfesten und axial verschiebbaren Eingriff mit einer nutförmigen Ausnehmung 13a einer Dosierhülse 13 stehen. Eine Drehung des Dosierglieds 3 relativ zu der Zeigeeinrichtung 4d bewirkt eine Drehung oder Mitnahme der Dosierhülse 13 in die entsprechende Drehrichtung.

Die Dosierhülse 13 weist ein Innengewinde 13c auf, welches in ein Außengewinde 8a eines hülsenförmigen Vortriebsglieds 8 eingreift, so dass die Dosierhülse 13 mittels einer Drehung relativ zu dem Vortriebsglied 8 an dem Vortriebsglied 8 entlang schraubbar ist.

Das hülsenförmige Vortriebsglied 8 weist eine das Außengewinde 8a überlagernde sich in Längsrichtung L erstreckende Nut 8b auf, in welche ein von dem Gehäuse 4 gebildetes Führungseingriffsglied 4g eingreift, so dass das Vortriebsglied 8 relativ zu dem Gehäuse 4 verdrehfest und axial verschiebbar ist. Das Gehäuse 4 weist an seiner als Innenhülse gebildeten Struktur, welche auch das Führungseingriffsglied 4g aufweist, eine Führung 4f auf, welche das Vortriebsglied 8, insbesondere im Bereich seines Außengewindes 8 führt, insbesondere seitlich führt. Ferner weist diese innenhülsenförmige Struktur ein federnd angeordnetes Halteeingriffsglied 4h auf, welches mit einem Federarm an dem Führungseingriffsglied 4g befestigt ist. Die von der Rücksetzfeder 12 in ihrer Halteposition gehaltenen Klemmhülse 16 hält das Halteeingriffglied 4h in solch einem Eingriff mit dem Vortriebsglied 8, dass das Vortriebsglied 8 relativ zu dem Halteeingriffsglied 4h gegen eine Verschiebung in distale Richtung entlang der Längsachse L gesperrt ist. Die Klemmhülse 16 weist eine Fläche, insbesondere eine konische Fläche auf, welche das Halteeingriffglied 4h in dem Halteeingriff mit dem Vortriebsglied 8 hält. Zum Beispiel kann der Nutgrund der Nut 8b eine Verzahnung (nicht gezeigt bzw. 8c in den anderen Ausführungsformen) aufweisen, in die das Halteeingriffsglied 4h eingreift.

An dem hülsenförmigen Vortriebsglied 8 stützt sich eine als Wendelfeder gebildete und als Druckfeder wirkende Ausschüttfeder 11 ab, insbesondere mit ihrem distalen Ende, welche so stark vorgespannt ist, dass die in ihr gespeicherte Energie ausreicht, das gesamte mittels Verschiebung des Kolbens aus dem Produktbehälter 14 ausschüttbare Produkt auszuschütten, insbesondere in mehreren Einzelausschüttungen. Das proximale Ende der Ausschüttfeder 11 stützt sich an dem Dosierglied 3 ab.

Die Dosierhülse 13 weist insbesondere an ihrem proximalen Ende eine erste Kupplungsstruktur 13b in der Gestalt einer über den Umfang angeordneten Verzahnung, insbesondere Außenverzahnung, auf. Das Rotationsglied 1 weist insbesondere an seinem distalen Ende eine zweite Kupplungsstruktur 1b auf, in welche die erste Kupplungsstruktur 13b in einen verdrehfesten Eingriff bringbar ist, wodurch eine Kupplung 1b, 13b, insbesondere eine Anzeigerücksetzkupplung, gebildet wird. Ist die Kupplung 1b, 13b geschlossen, ist das Rotationsglied 1 relativ zu der Dosierhülse 13 drehfest, wobei das Dosierglied 1 relativ zu der Dosierhülse 13 drehbar ist, wenn die Kupplung 1b, 13b geöffnet ist.

Das Gehäuse 4, insbesondere deren Innenhülse, bildet einen Dosieranschlag 4k für die Dosierhülse 13, die insbesondere mit ihrer distalen Stirnseite an dem Dosieranschlag 4k anschlagen kann.

Zwischen dem Gehäuse 4 und dem Dosisanzeigeelement 10 ist eine Anzeigerücksetzfeder 18 angeordnet, welche als Wendelfeder ausgestaltet ist und in der ersten Ausführungsform als Druckfeder wirkt. Die Steigung der Gewinde 9a, 10d ist so groß, dass keine Selbsthemmung zwischen dem Dosisanzeigeelement 10 und dem Lagerelement 9 stattfindet, wenn das Dosisanzeigeelement 10 mittels der Rücksetzfeder 18 mit einer entlang der Längachse L wirkenden Kraft beaufschlagt wird.

Das Gehäuse 4 weist an seinem proximalen Ende eine Verzahnung 4a auf, in welche ein Nocken 15a einer Rutschkupplungsfeder 15 eingreift, die drehfest mit dem Dosierglied 3 verbunden ist. Die Rutschkupplungsfeder 15 ist ringförmig, insbesondere ein Metallteil, welches zum Beispiel durch Stanzbiegen hergestellt wurde. Bei Drehung des Dosierglieds 3 relativ zu dem Gehäuse 4 wird der Nocken 15a über die Zähne der Verzahnung 4a bewegt, wodurch ein hörbares Geräusch während der Dosiseinstellung erzeugt wird. Zusätzlich kann der Eingriff des Nockens 15a in die Verzahnung 4a eine Rutschkupplung, insbesondere eine Zwei-Weg-Rutschkupplung bilden, welche verhindert, dass das Dosierglied 3 aufgrund der auf das Dosierglied 3 einwirkenden Federkräfte sich unbeabsichtigt dreht.

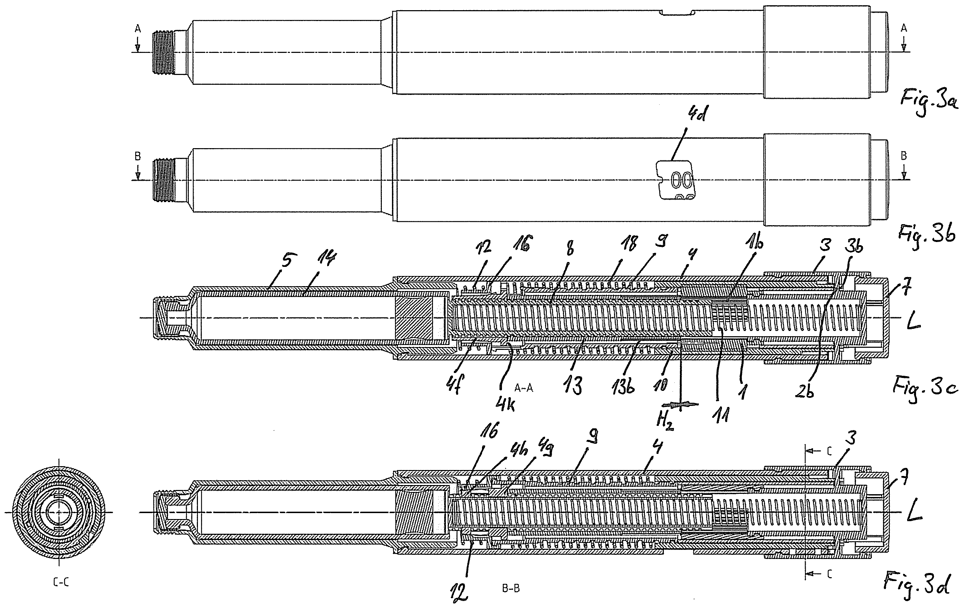

In den Figuren 3a bis 3d wird die Injektionsvorrichtung in einem Ausgangszustand gezeigt, wobei das Dosisanzeigeelement 10 seine Ausgangs- oder Nulldosisposition einnimmt, so dass in der Zeigeeinrichtung 4d die Dosis "00" ablesbar ist. Das Betätigungsglied 7 ist unbetätigt. Die aus den dritten und vierten Kupplungsstrukturen 2b, 3b gebildete Kupplung 2b, 3b ist geschlossen. Das Halteeingriffsglied 4h wird von der Klemmhülse 16 im Halteeingriff mit dem Vortriebsglied 8 gehalten.

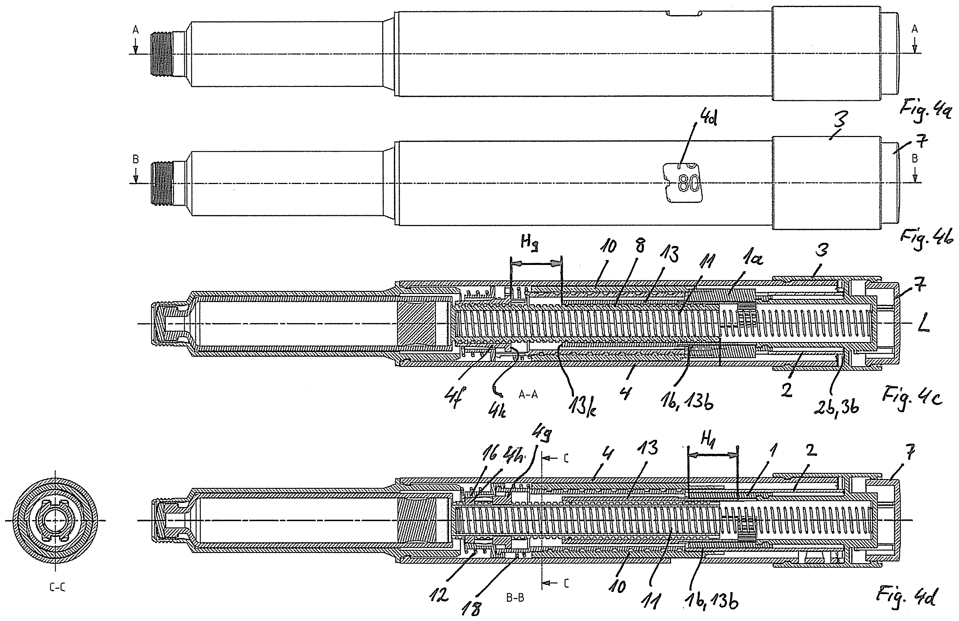

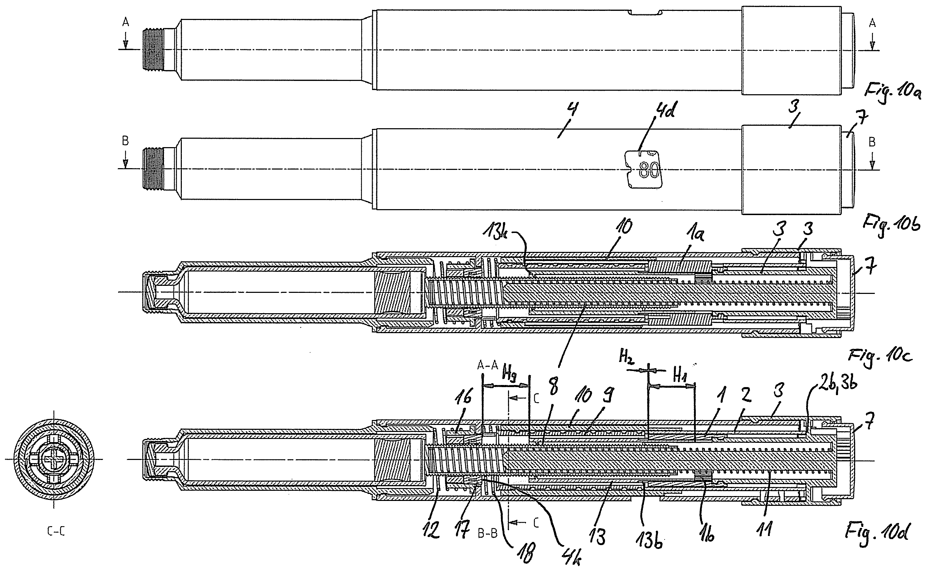

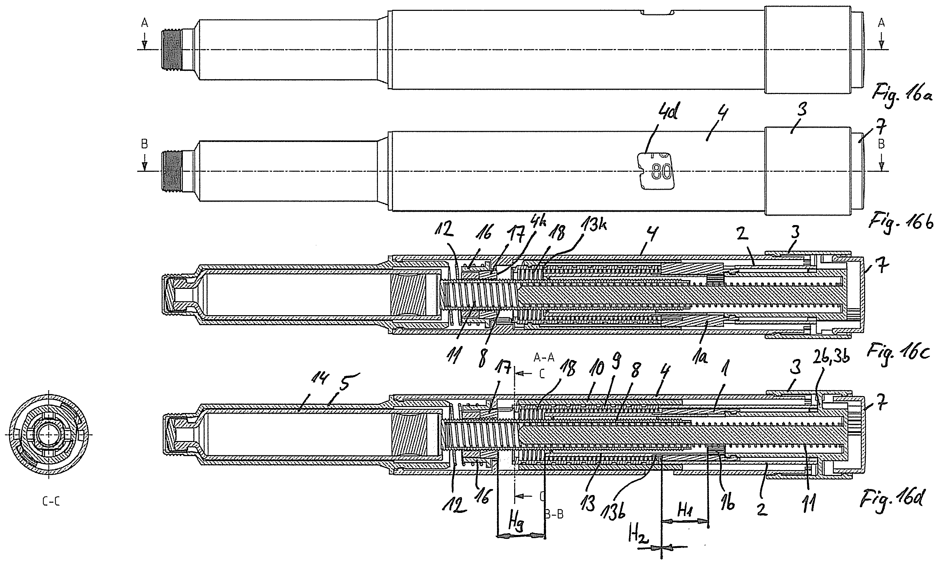

Zur Erhöhung der zu verabreichenden Produktdosis wird das Dosierglied 3 relativ zu dem Gehäuse 4 oder der Zeigeeinrichtung 4d in eine erste Drehrichtung gedreht, wobei mittels der geschlossenen Kupplung 2b, 3b das Kupplungsglied 2 mit dem Dosierglied 3 in die erste Drehrichtung gedreht wird. Das Kupplungsglied 2 dreht das Rotationsglied 1, wodurch sich das Dosisanzeigeelement 10 an dem relativ zu dem Gehäuse 4 drehfesten Lagerelement 9 entlang schraubt und die Anzeigerücksetzfeder 18 gespannt wird. Die Drehung des Dosierglieds 3 in die erste Drehrichtung bewirkt ferner eine Drehung der Dosierhülse 13 ebenfalls in die erste Drehrichtung, wodurch sich die Dosierhülse 13 an dem Vortriebsglied 8 in proximale Richtung entlang schraubt, wobei zwischen dem Dosieranschlag 4k und der Dosierhülse 13 ein Abstand gebildet wird, welcher einem Gesamtausschütthub H_{g} (Figuren 4c; 10c; 16c) entspricht. In der in Figur 3c gezeigten Position der Dosierhülse 13, insbesondere wenn die Dosis Null oder eine sehr geringe Dosis, wie zum Beispiel 1 oder 2 IU eingestellt ist, ist die Kupplung 1b, 13b geöffnet. Wird das Dosisanzeigeelement 10 aus seiner Nulldosisposition herausgedreht, insbesondere über die Dosis 1 oder 2 IU gedreht, wird die erste Kupplungsstruktur 13b in einen Eingriff mit der zweiten Kupplungsstruktur 1b verschoben, so dass die Kupplung 1b, 13b geschlossen ist.

Wenn die Dosierhülse 13 an den Dosieranschlag 4k anschlägt, insbesondere wenn die Dosis Null eingestellt wird oder eine Produktausschüttung stattgefunden hat, besteht zwischen der zweiten Kupplungsstruktur 1b und der ersten Kupplungsstruktur 13b entlang der Längsachse L ein einem zweiten Teilausschütthub H₂ entsprechender Abstand, der mitunter sehr klein sein kann, wie zum Beispiel nur wenige Hundertstel oder Zehntel Millimeter betragen kann. Wichtig ist lediglich, dass der Abstand so groß ist, dass die Kupplung 1b, 13b sicher geöffnet ist, wenn die Dosierhülse 13 an dem Dosieranschlag 4k anschlägt.

Durch Drehung des Dosierglieds 3 in die erste Drehrichtung wird - wie gesagt - der dem Gesamtausschütthub H_{g} entsprechende Abstand zwischen dem Dosieranschlag 4k und der Dosierhülse 13 gebildet, wobei die erste Kupplungsstruktur 13b mit der zweiten Kupplungsstruktur 1b entlang der Längsachse L um ein Maß überlappt, welches einem ersten Teilausschütthub H₁ entspricht. Während der zweite Teilausschütthub H₂ konstant ist, ist der erste Teilausschütthub H₁ variabel und abhängig von der eingestellten Dosis. Allgemein gilt: H_{g} > H₁; insbesondere gilt auch: H_{g} - H₁= H₂ ≠ 0 mm.

In dem in den Figuren 4a bis 4d gezeigten Zustand wird die Injektionsvorrichtung in einer Maximaldosisposition des Dosisanzeigeelements 10 gezeigt, wobei in dem Beispiel die maximale Dosis von 80 IU in der Zeigeeinrichtung 4d ablesbar ist. Durch Drehen des Dosierglieds 3 in die zweite, der ersten Drehrichtung entgegen gesetzten Drehrichtung kann die eingestellte Dosis verringert oder korrigiert werden, wobei der Abstand der Dosierhülse 13 zu dem Dosieranschlag 4k und/oder das Maß, mit dem sich die ersten und zweiten Kupplungsstrukturen 1b, 13b überlappen, verringert werden.

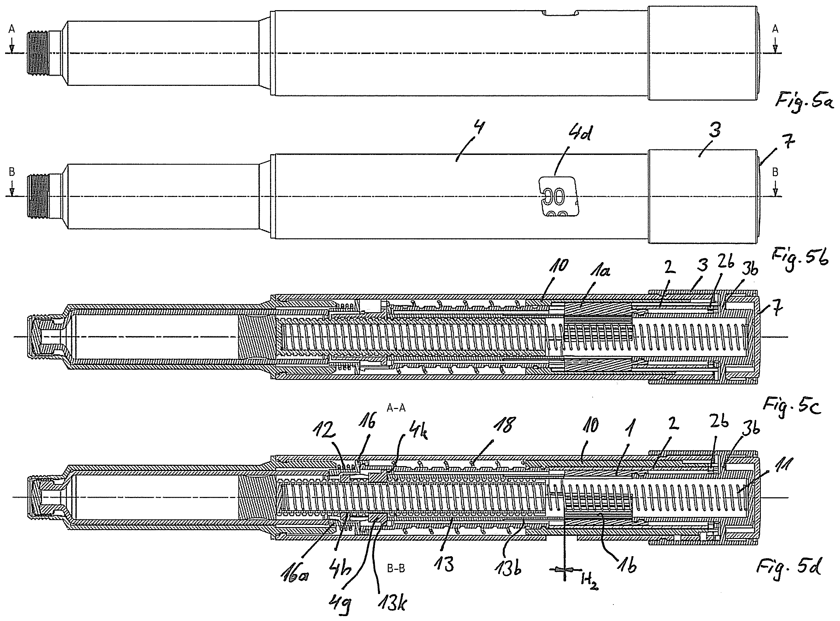

Zur Ausschüttung der eingestellten Produktdosis wird das Betätigungsglied 7 betätigt, insbesondere gedrückt (Figuren 5a bis 5d) wobei das mindestens eine Rastglied 7a des Betätigungsglieds 7 das Kupplungsglied 2 in distale Richtung verschiebt, wodurch die Kupplung 2b, 3b geöffnet wird. Das Kupplungsglied 2 nimmt das Lagerelement 9 mit, wodurch die Klemmhülse 16 gegen die Kraft der Rücksetzfeder 12 in distale Richtung verschoben wird. Durch die Verschiebung der Klemmhülse 16 ist das Halteeingriffsglied 4h freigegeben, so dass der axialfeste Halteeingriff zwischen dem Halteeingriffsglied 4h und dem Vortriebsglied 8 gelöst ist, wodurch die vorgespannte Feder 11 das Vortriebsglied 8 in distalen Richtung oder Ausschüttrichtung um den Gesamtausschütthub H_{g} verschiebt, das heißt so weit verschiebt, bis die Dosierhülse 13 an dem Dosieranschlag 4k anschlägt. Während des Gesamtausschütthubs H_{g} des Vortriebsglieds 8 bewegt sich die Dosierhülse 13 zunächst um den ersten Teilausschütthub H₁, wobei während der ersten Teilausschütthubs H₁ die Kupplung 1b, 13b geschlossen ist, so dass das Rotationsglied 1 relativ zu der Dosierhülse 13 drehfest ist. Dadurch, dass das Rotationsglied 1 drehfest mit dem Dosisanzeigeelement 10 verbunden ist, ist das Dosisanzeigeelement 10 in Bezug auf die Zeigeeinrichtung 4d drehfest. Am Ende des Teilausschütthubs H₁ wird die Kupplung 1b, 13b geöffnet, wobei die Dosierhülse 13 dann ihren zweiten Teilausschütthub H₂ ausführt und schließlich an dem Dosieranschlag 4k anschlägt. Sobald die Kupplung 1b, 13b geöffnet ist, ist das Rotationsglied 1 nicht mehr verdrehgesichert in Bezug auf das Gehäuse 4 bzw. die Zeigeeinrichtung 4d, wodurch das Dosisanzeigeelement 10 aufgrund der vorgespannten Rücksetzfeder 18 an dem Lagerelement 9 schlagartig in ihre Ausgangs- oder Nulldosisposition zurückgeschraubt wird (Figuren 5a bis 5d), wobei das Dosisanzeigeelement mit seinem Nulldosisanschlag 10c an dem Nulldosisgegenanschlag 4c anschlägt.

Bei Erreichen seiner Ausgangs- oder Nulldosisposition löst das Dosisanzeigeelement 10 mittels der Verzahnung 10b den das Dosierglied 7 in der betätigten Position haltenden Rasteingriff des mindestens einen Rastglieds 7a, wodurch die Rücksetzfeder 12 das Betätigungsglied 7 in seine unbetätigten Position zurücksetzt. Beim Rücksetzen des Betätigungsglieds 7 in seine unbetätigte Position verschiebt die Rücksetzfeder 12 die Klemmhülse 16 in ihre Klemm- oder Halteposition, in der die Klemmhülse 16 das Halteeingriffsglied 4h in einen axialfesten Eingriff mit dem Vortriebsglied 8 drückt. Gleichzeitig wird das Lagerelement 9 zusammen mit dem Dosisanzeigelement 10 in proximale Richtung verschoben oder zurückgesetzt. Ferner wird das Kupplungsglied 2 zurückgesetzt, wodurch die Kupplung 2b, 3b geschlossen wird.

Die Vorrichtung ist nun bereit für eine weitere Dosiseinstellung. Durch wiederholtes Einstellen und Ausschütten der Dosis kann das in dem Produktbehälter 14 enthaltene Produkt mit mehreren Gesamtausschütthüben oder mehreren beliebig wählbaren Einzeldosen ausgeschüttet werden.

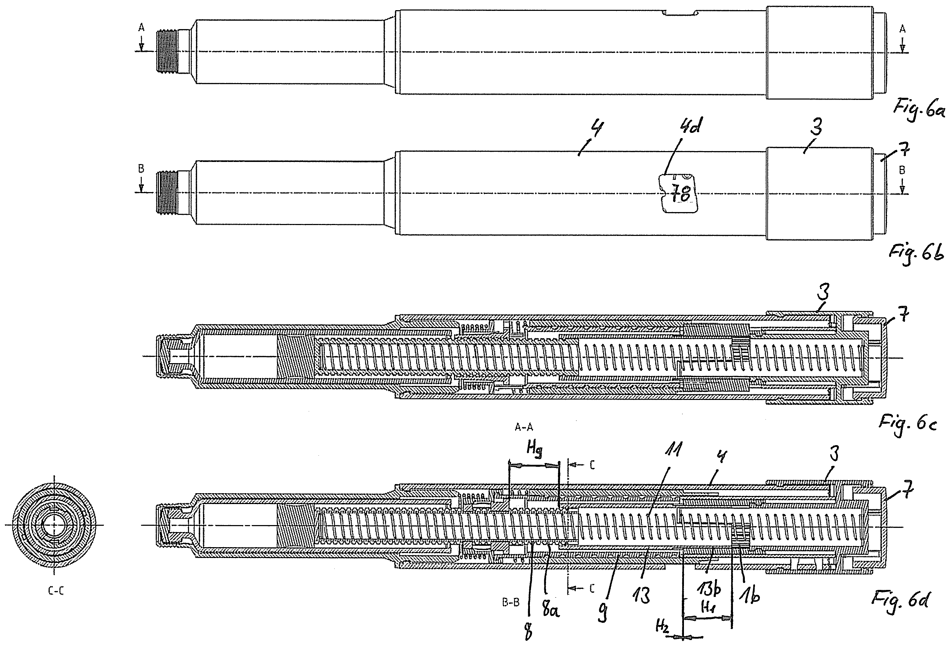

In den Figuren 6a bis 6d wird der Zustand der Antriebs- und Dosiervorrichtung dargestellt, bei der in dem Produktbehälter 14 eine ausschüttbare Menge des Produkts enthalten ist, die geringer als die mit der Antriebs- und Dosiervorrichtung einstellbare Maximaldosis ist. In dem gezeigten Beispiel sind 78 IU in dem Produktbehälter 14 enthalten, wobei mit der Antriebs- und Dosiervorrichtung maximal 80 IU einstellbar wären. Um eine Fehlanwendung zu vermeiden, umfasst die Antriebs- und Dosiervorrichtung eine Begrenzungseinrichtung, welche die Dosiseinstellung begrenzt. Hierzu weist das Vortriebsglied 8 einen Stoppanschlag am proximalen Ende des Gewindes 8a auf, an dem die Dosierhülse 13 anschlägt, wodurch eine Drehung des Dosierglieds 3 in die erste Drehrichtung blockiert wird, auch wenn ein optional vorhandener Maximaldosisanschlag nicht in einem Kontakt mit einem entsprechenden Maximaldosisgegenanschlag ist. Eine Drehung des Dosierglieds 3 in die zweite Drehrichtung, das heißt in eine Drehrichtung, die ein Abdosieren oder eine Verringerung der Dosis bewirkt, ist jedoch möglich.

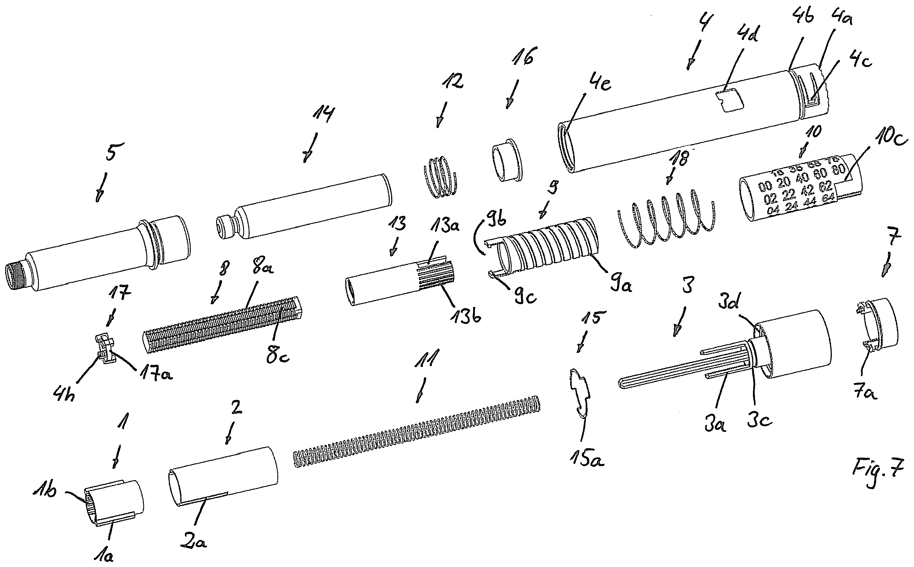

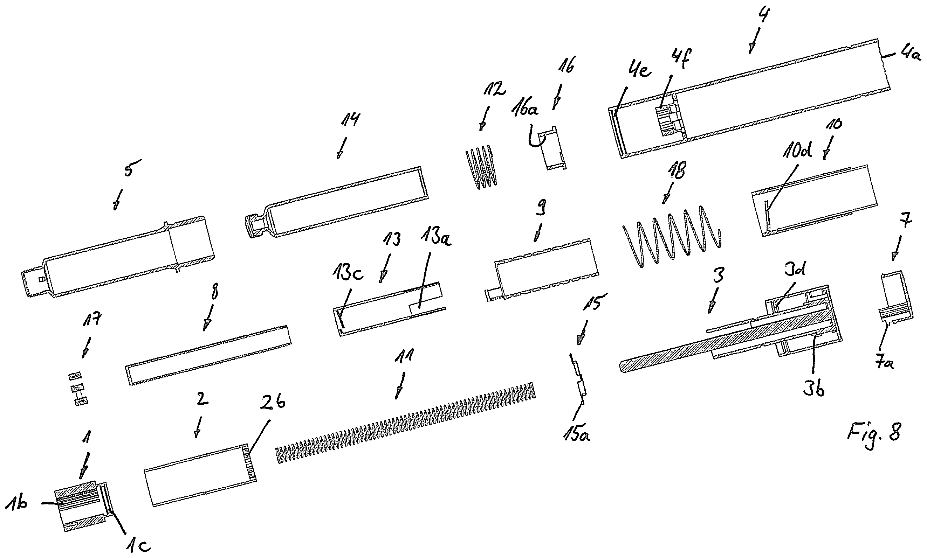

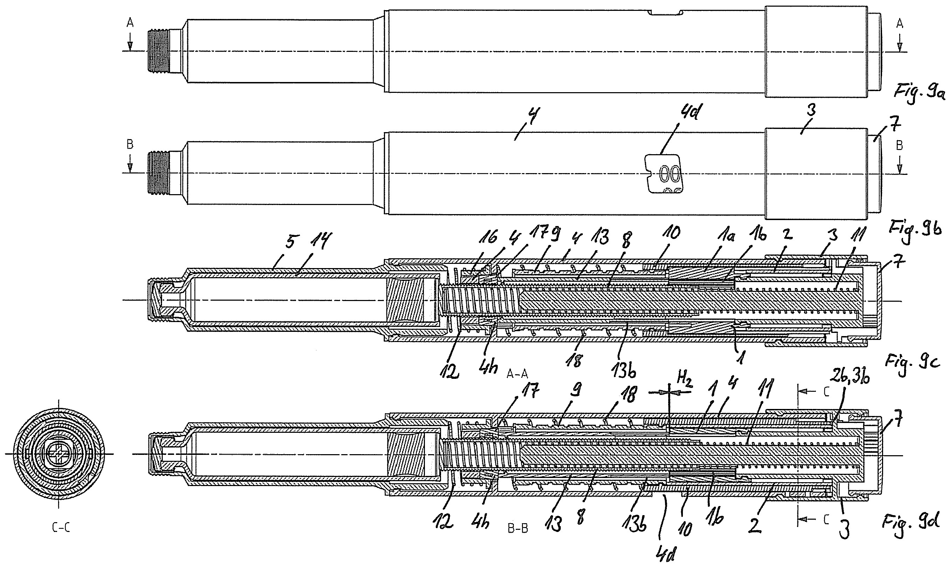

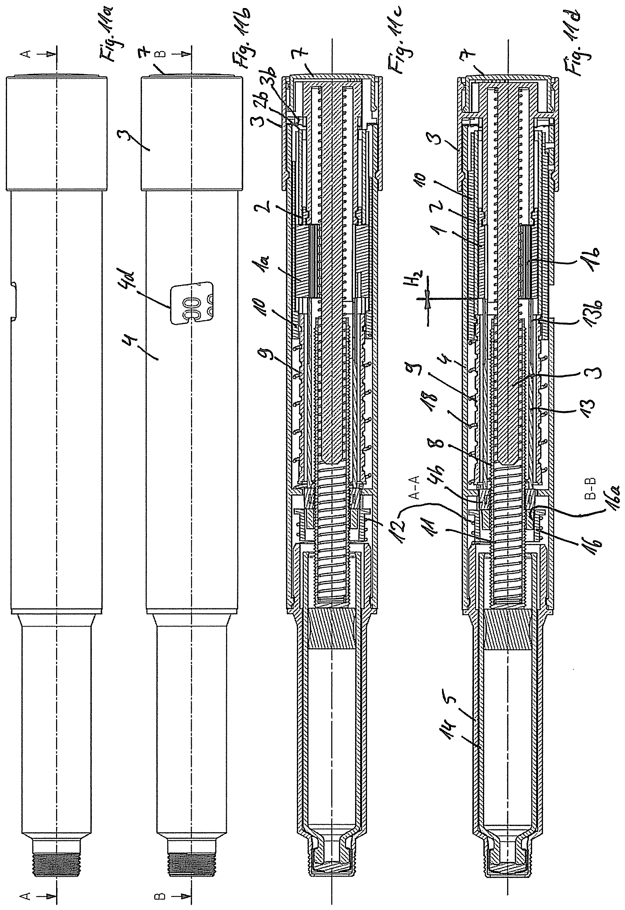

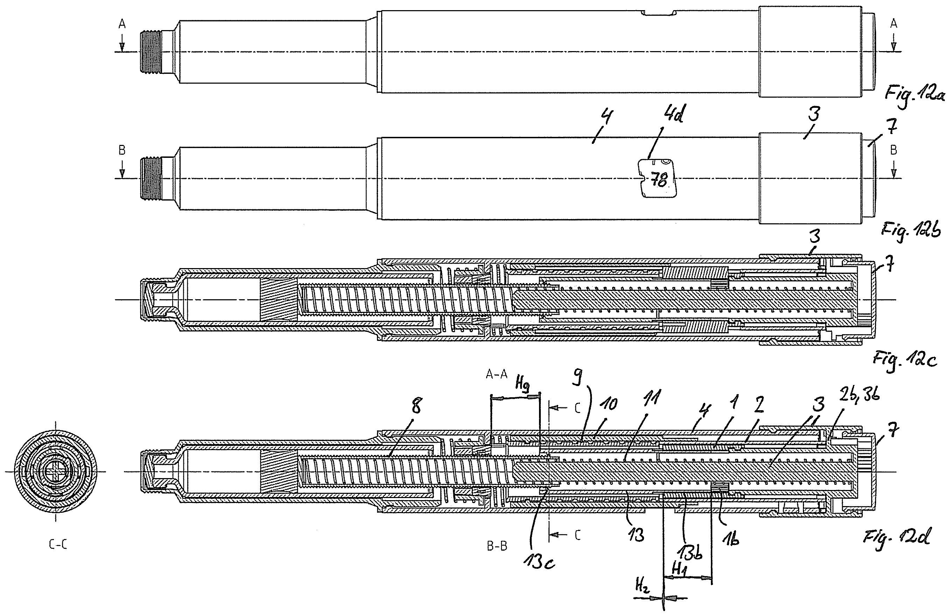

In der zweiten, in den Figuren 7 bis 12d gezeigten Ausführungsformen ist das Betätigungsglied 7 so ausgestaltet, dass es für die Produktausschüttung vom Verwender in der betätigten Position gehalten werden muss, das heißt nicht mit dem Dosierglied 3 in einer betätigten Position verrastet. Hierdurch wird bewirkt, dass der Verwender der Vorrichtung den Injektionsvorgang unterbrechen kann. Allerdings ist es alternativ selbstverständlich möglich, bei der zweiten Ausführungsform einen Mechanismus, der das Dosierglied 7 in seiner betätigten Position hält vorzusehen, wie zum Beispiel den Mechanismus aus der ersten Ausführungsform.

Das Dosierglied 3 weist ein stabförmiges Führungsmittel auf, welches von der Ausschüttfeder 11 umgeben wird und ein seitliches Ausknicken der Ausschüttfeder 11 verhindert. Dieses Führungsmittel kann auch für die anderen hierin beschriebenen Ausführungsformen vorgesehen sein.

Abweichend von der ersten Ausführungsform ist das mindestens eine Halteeingriffsglied 4h nicht von dem Gehäuse 4 sondern von einem axialfest in dem Gehäuse 4 aufgenommenen Greifring 17 gebildet, der in dem gezeigten Beispiel vier Halteeingriffsglieder 4h und eine Ringfeder 17a aufweist, wobei die Halteeingriffsglieder 4h über den Umfang der Ringfeder 17a verteilt an der Ringfeder 17a befestigt sind. Die Ringfeder 17a kann zum Beispiel aus Metall oder Kunststoff sein. Der Greifring 17 einschließlich der Ringfeder 17a und der Halteeingriffsfeder 4h kann z.B. ein einteilig hergestelltes Kunststoffspritzgussteil sein.

Alternativ kann eine aus Metall gebildete Ringfeder 17a vorgesehen sein, die zum Beispiel mittels eines Kunststoffspritzgussverfahrens mit einem oder mehreren, wie zum Beispiel zwei oder vier Halteeingriffsgliedern 4h umspritzt wird.

Die Klemmhülse 16 weist an ihrem Innenumfang eine konische Fläche 16a auf, welche bewirkt, dass die zwischen der Halte- oder Klemmposition und der Freigabeposition hin und her verschiebbare Klemmhülse 16 die Halteeingriffsglieder 4h in den Eingriff mit einer sich entlang der Längsachse erstreckenden Verzahnung 4c des Vortriebsglieds 8 drückt oder zum Lösen aus der Verzahnung 8c freigibt.

Im Übrigen wird auf die Beschreibung zur ersten Ausführungsform verwiesen.

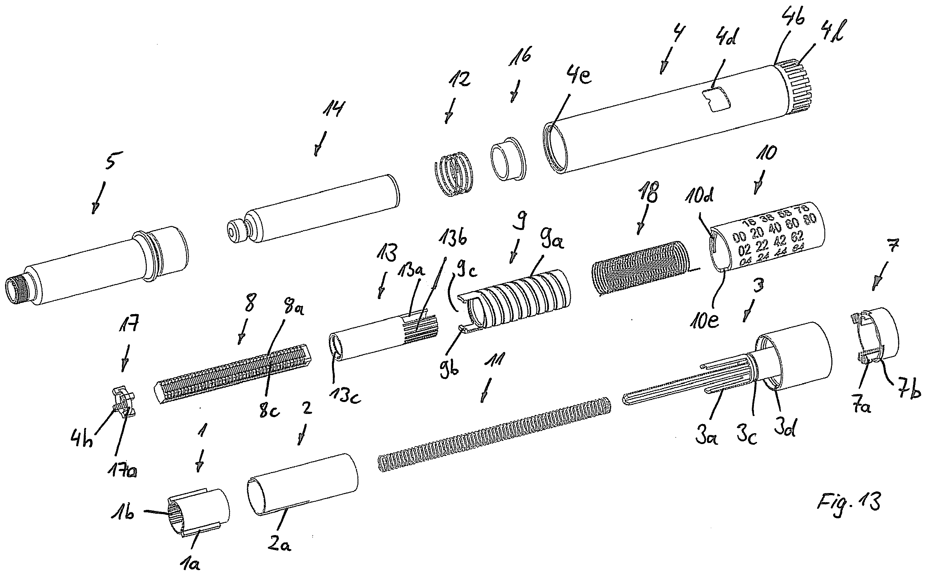

Die dritte Ausführungsform aus den Figuren 13a bis 13d ist ähnlich aufgebaut wie die zweite Ausführungsform und im weitesten Sinne auch wie die erste Ausführungsform.

Die dritte Ausführungsform unterscheidet sich im Wesentlichen dadurch von der ersten Ausführungsform, dass statt der als Druckfeder wirkenden Anzeigerücksetzfeder 18 eine als Drehfeder wirkende und als Wendelfeder ausgestaltete Anzeigerücksetzfeder 18 für das Rücksetzen des Dosisanzeigeelements 10 in seine Ausgangs- oder Nulldosisposition vorgesehen ist. Hierzu kann das Gewinde 9a optional aber nicht notwendigerweise mit einer so geringen Steigung ausgestaltet sein, dass eine Selbsthemmung bei einer axialen Belastung auftreten würde.

Die Anzeigerücksetzfeder 18 stützt sich mit einem Ende drehfest an dem Rotationsglied 1, alternativ an dem Dosisanzeigeelement 10, und mit dem anderen Ende drehfest an dem Gehäuse 4 ab. Eine Drehung des Dosierglieds 3 in die erste Drehrichtung spannt die Anzeigerücksetzfeder 18, wobei eine Drehung des Dosierglieds 3 in die zweite Drehrichtung die Anzeigerücksetzfeder 18 entspannt.

Das Dosisanzeigeelement 10 zeigt zwar keinen Nulldosisanschlag, kann jedoch einen Nulldosisanschlag, wie er zum Beispiel in der ersten und zweiten Ausführungsform gezeigt ist, aufweisen. Das Dosisanzeigeelement 10 weist einen Maximaldosisanschlag 10e auf, der, wenn eine Maximaldosis eingestellt ist, wie zum Beispiel hier 80 IU, an einen Maximaldosisgegenanschlag 4i anschlägt, der z.B. von dem Gehäuse 4 gebildet wird. Eine Drehung des Dosierglieds 3 in die erste Drehrichtung wird dann blockiert, wobei eine Verringerung der Dosis, das heißt eine Drehung des Dosierglieds 3 in die zweite Drehrichtung möglich ist. Obwohl nicht gezeigt, kann das Dosisanzeigeelement 10 der ersten und zweiten Ausführungsform einen solchen Maximaldosisanschlag 10e bzw. Maximaldosisgegenanschlag 4i aufweisen, insbesondere alternativ oder zusätzlich zu dem Nulldosisanschlag 10c.

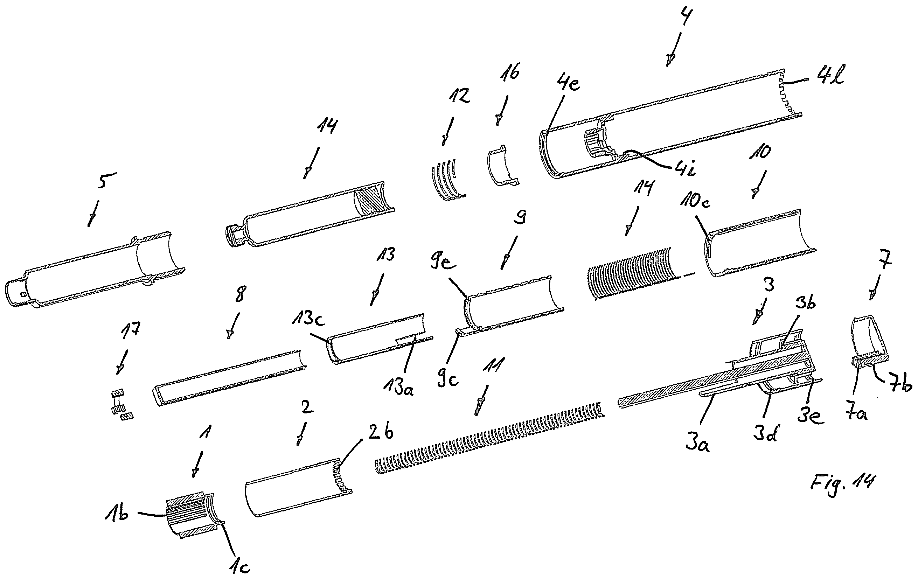

In den Figuren 13 und 14 wird am proximalen Ende des Gehäuses 4 eine Verzahnung 41 gezeigt, in welche das Rastglied 7a durch Betätigung des Betätigungsglieds 7 eingreift oder einkuppelbar ist, so dass das Betätigungsglied 7 und das Dosierglied 3 bei betätigtem Beätigungsglied 7 relativ zu dem Gehäuse 4 verdrehgesichert sind. Das Betätigungsglied 7 weist einen Ringsteg 7b auf, welcher bei der Montage über einen Ringsteg 3e des Dosierglieds 3 geschnappt wird und dann axial an den Ringsteg 3e anschlägt, um ein Herausfallen des Betätigungsglieds 7 aus dem Dosierglied 3 zu verhindern.

**Bezugszeichenliste**

| | | | |
|---|---|---|---|
| 1 | Rotationsglied | 9c | Steg |
| 1a | Steg | 9e | Schulter |
| 1b | zweite Kupplungsstruktur | | |
| 1c | Ringsteg | 10 | Dosisanzeigeelement / Dosisanzeigetrommel |
| 2 | Kupplungsglied | 10a | Dosisskala |
| 2a | Ausnehmung/Nut | 10b | Zähne/Verzahnung |
| 2b | dritte Kupplungsstruktur/Verzahnung | 10c | Nulldosisanschlag |
| | | 10d | Innengewinde |
| | | 10e | Maximaldosisanschlag |
| 3 | Dosierglied | | |
| 3a | Steg | 11 | Antriebsfeder |
| 3b | vierte Kupplungsstruktur | | |
| 3c | Ringnut | 12 | Rücksetzfeder |
| 3d | Ringsteg | | |
| 3e | Ringsteg | 13 | Dosierhülse |
| | | 13a | Ausnehmung/Nut |
| 4 | Gehäuse | 13b | erste |
| 4a | Verzahnung | | Kupplungsstruktur/Verzahnung |
| 4b | Ringnut | 13c | Innengewinde |
| 4c | Nulldosisgegenanschlag | 13k | Dosiergegenanschlag / |
| 4d | Zeigeeinrichtung | | Stirnfläche |
| 4e | Ringsteg | | |
| 4f | Führung | 14 | Produktbehälter |
| 4g | Führungseingriffsglied | | |
| 4h | Halteeingriffsglied | 15 | Rutschkupplungsfeder/Zwei-Weg-Rutschkupplung |
| 4i | Maximaldosisgegenanschlag | | |
| 4j | Führungssteg | 15a | Nocken |
| 4k | Dosieranschlag | | |
| 4l | Verzahnung | 16 | Klemmstück / Klemmhülse |
| | | 16a | Konus |
| 5 | Produktbehälterhalter | 16b | Nut |
| 7 | Betätigungsglied | 17 | Greifring |
| 7a | Rastglied | 17a | Ringfeder |
| 7b | Ringsteg | 18 | Rücksetzfeder / Anzeigerücksetzfeder |
| 8 | Vortriebsglied | | |
| 8a | Außengewinde | | |
| 8b | Nut | L | Längsachse / Drehachse |
| 8c | Zahnstruktur | H₁ | erster Teilausschütthub |
| | | H₂ | zweiter Teilausschütthub |
| 9 | Lagerelement | H_{g} | Gesamtausschütthub |
| 9a | Außengewinde | | |
| 9b | Ausnehmung | | |

## Patentansprüche

1. Antriebs- und Dosiervorrichtung für eine Injektionsvorrichtung zur Verabreichung eines flüssigen Produkts, wobei mit der Antriebs- und Dosiervorrichtung eine zu verabreichende Produktdosis einstellbar ist, umfassend:
a) ein Dosisanzeigeelement (10), über dessen Umfang eine Dosisskala (10b) angeordnet ist,
b) eine Zeigeeinrichtung (4d) und ein vom Verwender der Antriebs- und Dosiervorrichtung greifbares Dosierglied (3), wobei zur Einstellung der zu verabreichenden Dosis durch Drehung des Dosierglieds (3) relativ zu der Zeigeeinrichtung (4d) das Dosisanzeigeelement (10) relativ zu der Zeigeeinrichtung (4d) um eine Drehachse (L) drehbar ist und mittels der Zeigeeinrichtung (4d) ein Wert der Dosisskala (10b) ablesbar ist, welcher der eingestellten Dosis entspricht,
c) eine Ausschüttfeder (11) und ein Vortriebsglied (8), wobei die Ausschüttfeder (11) die für die Ausschüttung des Produkts erforderliche Energie speichert und für die Produktausschüttung an das Vortriebsglied (8) abgibt, wodurch das Vortriebsglied (8) um einen Gesamtausschütthub (H_{g}) in Vortriebsrichtung bewegt wird,
**dadurch gekennzeichnet, dass**
d) der Gesamtausschütthub (H_{g}) einen ersten Teilausschütthub (H₁) umfasst, während dem das Dosisanzeigeelement (10) in Bezug auf die Zeigeeinrichtung (4d) drehfest ist, und dass der Gesamtausschütthub (H_{g}) einen von der eingestellten Dosis unabhängigen zweiten Teilausschütthub (H₂) umfasst, wobei das Dosisanzeigeelement (10) nach dem ersten Teilausschütthub (H₁) während dem zweiten Teilausschütthub (H₂) relativ zu der Zeigeeinrichtung (4d) drehbar und aufgrund einer vorgespannten Rücksetzfeder (18) in eine Nulldosisposition zurückschraubbar ist, und dass
e) die Vorrichtung eine Kupplung (1b, 13b) umfasst, welche während des ersten Teilausschütthubs (H₁) im geschlossenen Zustand die Zeigeeinrichtung (4d) oder das Gehäuse (4) drehfest mit dem Dosisanzeigeelement (10) koppelt, und im geöffneten Zustand die Zeigeeinrichtung (4d) oder das Gehäuse (4) von dem Dosisanzeigeelement (10) rotatorisch entkoppelt, so dass das Dosisanzeigeelement (10) relativ zu der Zeigeeinrichtung (4d) drehbar ist.

2. Antriebs- und Dosiervorrichtung nach Anspruch 1, **gekennzeichnet durch** ein Lagerelement (9), welches in Bezug auf das Gehäuse (4) drehfest und axial verschiebbar ist und ein Gewinde (9a) aufweist, in welches das Dosisanzeigeelement (10) eingreift, so dass das Dosisanzeigeelement (10) relativ zu dem Lagerelement (9) schraubbar ist.

3. Antriebs- und Dosiervorrichtung nach dem vorhergehenden Anspruch, **gekennzeichnet durch** ein vom Verwender der Antriebs- und Dosiervorrichtung für die Produktausschüttung betätigbares Betätigungsglied (7), dessen Betätigung bewirkt, dass das Lagerelement (9) relativ zu der Zeigeeinrichtung (4d) entlang der Längsachse (L) verschoben wird.

4. Antriebs- und Dosiervorrichtung nach einem der vorhergehenden Ansprüche, **dadurch gekennzeichnet, dass** die Kupplung (1b, 13b) eine erste Kupplungsstruktur (13b), welche von einer in Bezug auf das Dosierglied (3) drehfesten und axial verschiebbaren Dosierhülse (13) gebildet wird, und eine zweite Kupplungsstruktur (1b), welche von einem drehfest und axial verschiebbar mit dem Dosisanzeigeelement (10) verbundenen Rotationsglied (1) gebildet ist, aufweist, wobei nach erfolgter Dosiseinstellung die erste Kupplungsstruktur (13b) mit der zweiten Kupplungsstruktur (1b) entlang der Drehachse (L) um ein Maß überlappt, welches dem ersten Teilausschütthub H₁ entspricht.

5. Antriebs- und Dosiervorrichtung nach einem der vorhergehenden Ansprüche, **dadurch gekennzeichnet, dass** die Ausschüttfeder (11) so stark vorgespannt ist, dass die in ihr gespeicherte Energie ausreicht, das in einem Produktbehälter (14) enthaltene Produkt in mehreren Einzelausschüttungen auszuschütten.

6. Antriebs- und Dosiervorrichtung nach einem der vorhergehenden Ansprüche, **dadurch gekennzeichnet, dass** die Ausschüttfeder (11) eine Druckfeder, insbesondere eine als Druckfeder wirkende Wendelfeder ist.

7. Antriebs- und Dosiervorrichtung nach einem der vorhergehenden Ansprüche, **dadurch gekennzeichnet, dass** das Vortriebsglied (8) mittels einer Greifeinrichtung axial festgehalten wird, die vorzugsweise in ein Außengewinde (8a) des in Bezug auf das Gehäuse (4) verdrehgesicherten und axial verschiebbaren Vortriebsglieds (8) eingreift, wobei der Eingriff der Greifeinrichtung in das Vortriebsglied (8) durch Betätigung eines Betätigungsglieds (7) lösbar ist, wodurch das Vortriebsglied (8) in distale Richtung oder Ausschüttrichtung verschiebbar ist.

8. Antriebs- und Dosiervorrichtung nach einem der vorhergehenden Ansprüche, **gekennzeichnet durch** eine Dosierhülse (13), welche in ein Außengewinde (8a) des Vortriebsglieds (8) eingreift und durch Drehung eines Dosierglieds (3) relativ zu dem Vortriebsglied (8) schraubbar ist, wobei mittels Drehung des Dosierglieds (3) in eine Drehrichtung, welche eine Dosiserhöhung bewirkt, ein Dosierabstand zwischen der Dosierhülse (13) und einem Dosieranschlag (4k) erhöht wird.

9. Antriebs- und Dosiervorrichtung nach dem vorhergehenden Anspruch, **dadurch gekennzeichnet, dass** der Abstand zwischen dem Dosieranschlag (4k) und der Dosierhülse (13) durch Drehung des Dosierglieds (3) in eine Drehrichtung, welche eine Dosisverringerung bewirkt, verringert wird.

10. Antriebs- und Dosiervorrichtung nach einem der zwei vorhergehenden Ansprüche, **dadurch gekennzeichnet, dass** der Abstand zwischen der Dosierhülse (13) und dem Dosieranschlag (4k) dem Gesamtausschütthub (H_{g}) des Vortriebsglieds (8) entspricht.

11. Antriebs- und Dosiervorrichtung nach dem vorhergehenden Anspruch, **dadurch gekennzeichnet, dass** das Betätigungsglied (7) bei seiner Betätigung mit dem Dosierglied (3) verrastet, wobei diese Rastverbindung mittels des Dosisanzeigeelements (10) lösbar ist.

12. Antriebs- und Dosiervorrichtung nach Anspruch 7, **dadurch gekennzeichnet, dass** die Greifeinrichtung ein Halteeingriffsglied (4h) umfasst, welches von dem Gehäuse (4) oder einem Greifring (17) gebildet wird.

## Claims

1. A drive and metering device for an injection device for dispensing a liquid product, wherein a product dose to be discharged can be set with the drive and metering device, comprising:
a) a dose display element (10) comprising a dosage scale (10b) arranged over its circumference;
b) an indicator (4b) and a metering member (3) that can be gripped by a user of the drive and metering device; wherein the dose display element (10) can be rotated relative to the indicator (4d) about a longitudinal axis (L) by rotating the metering member (3) relative to the indicator (4d) to set a dose to be administered such that a value of the dosage scale (10b) that corresponds to the set dose can be read via the indicator (4d),
c) a discharge spring (11) and an advancement member (8), wherein the discharge spring (11) stores the energy required to discharge the product and delivers the energy to the advancement member (8) for product discharge such that the advancement member (8) is moved by a total discharge stroke (H_{g}) in the discharge direction,
**characterized in that**
d) the total discharge stroke (H_{g}) comprises a first partial discharge stroke (H₁), during which the dose display element (10) is rotationally fixed relative to the indicator (4d), and the total discharge stroke (H_{g}) comprises a second partial discharge stroke (H₂) independent of the set product dose, wherein after the first partial discharge stroke (H₁) during the second partial discharge stroke (H₂) the dose display element (10) is rotatable relative to the indicator (4d) and can be screwed back into a zero-dose position by a preloaded reset spring (18), and **in that**
e) the drive and metering device comprises a clutch (1b, 13b), which in an engaged state, during the first partial discharge stroke (H₁), couples the indicator (4d) or a housing (4) of the drive and metering device rotationally fixedly to the dose display element (10), and which, in a disengaged state of the clutch, rotationally decouples the indicator (4d) or the housing (4) from the dose display element (10) such that the dose display element (10) is rotatable relative to the indicator (4d).

2. The drive and metering device of claim 1, **characterized by** a bearing element (9) rotationally fixed and axially movable relative to the housing (4), the bearing element comprising a thread (9a) engaged with the dose display element (10) such that the dose display element (10) can be screwed relative to the bearing element (9).

3. The drive and metering device of claim 2, **characterized by** an actuating element (7) that can be actuated by a user of the drive and metering device for discharge of the product by causing the bearing element (9) to be displaced relative to the indicator (4d) along the longitudinal axis (L).

4. The drive and metering device of one of the preceding claims, **characterized in that** the clutch (1b, 13b) comprises a first clutch structure (13b) formed by a metering sleeve (13) rotationally fixed and axially displaceable relative to the metering member (3), and a second clutch structure (1b) formed by a rotation element (1) coupled to the dose display element (10) for enabling conjoint rotation and axial movement, wherein when the product dose is set, the first clutch structure (13b) and the second clutch structure (1b) overlap along the longitudinal axis (L) to an extent corresponding to the first partial discharge stroke (H₁).

5. The drive and metering device of one of the preceding claims, **characterized in that** the discharge spring (11) is preloaded to the extent that the stored energy suffices to discharge the product contained in a product container (14) in a plurality of individual discharges.

6. The drive and metering device of one of the preceding claims, **characterized in that** the discharge spring (11) is a compression spring.

7. The drive and metering device of one of the preceding claims, **characterized in that** the advancement member (8) is coupled rotationally fixedly and axially movably to the housing (4) and is retained axially by a gripping device being in a releasable engagement with an external thread of the advancement member (8), wherein actuation of an actuating element (7) for actuating discharge of the product causes the engagement of the gripping device with the advancement member (8) to be released such that the advancement member (8) is movable in the discharge direction.

8. The drive and metering device of one of the preceding claims, **characterized by** a metering sleeve (13) configured to engage with an external thread (8a) of the advancement member (8), wherein the metering sleeve (13) can be screwed by rotating the metering member (3) relative to the advancement member (8), wherein a metering distance between the metering sleeve (13) and a metering stop (4k) is increased by rotating the metering member (3) in a rotational direction that causes an increase of the dose.

9. The drive and metering device of claim 8, **characterized in that** the metering distance between the metering stop (4k) and the metering sleeve (13) is reduced by rotating the metering member (3) in a rotational direction that causes a reduction of the dose.

10. The drive and metering device of claim 8 or 9, **characterized in that** the metering distance between the metering sleeve (13) and the metering stop (4k) corresponds to the total discharge stroke (H_{g}) of the advancement member (8).

11. The drive and metering device of claim 10, wherein upon actuation, the actuating element (7) releasably interlocks with the metering member (3), and wherein the interlocking can be released by the dose display element (10).

12. The drive and metering device of claim 7, wherein the gripping device comprises a retaining engagement element (4h) formed by the housing (4) or a gripping ring (17).

## Revendications

1. Dispositif d'entraînement et de dosage pour un dispositif d'injection pour l'administration d'un produit fluide, une dose de produit à administrer étant réglable avec le dispositif d'entraînement et de dosage, comprenant :
a) un élément d'affichage de dose (10), sur la circonférence duquel une échelle de dose (10b) est agencée,
b) un dispositif de pointage (4d) et un organe de dosage (3) pouvant être saisi par l'utilisateur du dispositif d'entraînement et de dosage, dans lequel l'élément d'affichage de dose (10) est rotatif autour d'un axe de rotation (L) par rapport au dispositif de pointage (4d) pour le réglage de la dose à administrer par rotation de l'organe de dosage (3) par rapport au dispositif de pointage (4d) et une valeur de l'échelle de dose (10b), laquelle correspond à la dose réglée, peut être lue au moyen du dispositif de pointage (4d),
c) un ressort de déversement (11) et un organe de propulsion (8), dans lequel le ressort de déversement (11) stocke l'énergie nécessaire au déversement du produit et la fournit à l'organe de propulsion (8) pour le déversement de produit, moyennant quoi l'organe de propulsion (8) est déplacé d'une course de déversement totale (H_{g}) dans la direction de propulsion,
**caractérisé en ce que**
d) la course de déversement totale (H_{g}) comprend une première course de déversement partielle (H₁), pendant laquelle l'élément d'affichage de dose (10) est solidaire en rotation par rapport au dispositif de pointage (4d), et que la course de déversement totale (H_{g}) comprend une deuxième course de déversement partielle (H₂) indépendante de la dose réglée, dans lequel l'élément d'affichage de dose (10) est rotatif par rapport au dispositif de pointage (4d) après la première course de déversement partielle (H₁) pendant la deuxième course de déversement partielle (H₂) et est dévissable dans une position de dose nulle en raison d'un ressort de rappel précontraint (18), et que
e) le dispositif comprend un accouplement (1b, 13b), lequel couple à l'état fermé pendant la première course de déversement partielle (H₁) le dispositif de pointage (4d) ou le boîtier (4) solidaire en rotation avec l'élément d'affichage de dose (10), et découple à l'état ouvert le dispositif de pointage (4d) ou le boîtier (4) en rotation de l'élément d'affichage de dose (10), de sorte que l'élément d'affichage de dose (10) est rotatif par rapport au dispositif de pointage (4d).

2. Dispositif d'entraînement et de dosage selon la revendication 1, **caractérisé par** un élément de palier (9), lequel est axialement coulissant et solidaire en rotation par rapport au boîtier (4) et présente un filetage (9a), dans lequel l'élément d'affichage de dose (10) vient en prise de sorte que l'élément d'affichage de dose (10) peut être vissé par rapport à l'élément de palier (9).

3. Dispositif d'entraînement et de dosage selon la revendication précédente, **caractérisé par** un organe d'actionnement (7) actionnable par l'utilisateur du dispositif d'entraînement et de dosage pour le déversement de produit, dont l'actionnement entraîne le fait que l'élément de palier (9) est coulissant le long de l'axe longitudinal (L) par rapport au dispositif de pointage (4d).

4. Dispositif d'entraînement et de dosage selon l'une quelconque des revendications précédentes, **caractérisé en ce que** l'accouplement (1b, 13b) présente une première structure d'accouplement (13b), laquelle est formée par une douille de dosage (13) axialement coulissante et solidaire en rotation par rapport à l'organe de dosage (3), et une deuxième de structure d'accouplement (1b), laquelle est formée par un organe de rotation (1) relié à l'élément d'affichage de dose (10) solidaire en rotation et axialement coulissant, dans lequel après le réglage de dose, la première structure d'accouplement (13b) chevauche la deuxième structure d'accouplement (1b) le long de l'axe de rotation (L) d'une mesure qui correspond à la première course de déversement partielle H₁.

5. Dispositif d'entraînement et de dosage selon l'une quelconque des revendications précédentes, **caractérisé en ce que** le ressort de déversement (11) est précontraint de sorte que l'énergie stockée dans celui-ci suffit à déverser le produit contenu dans un récipient de produit (14) en plusieurs déversements individuels.

6. Dispositif d'entraînement et de dosage selon l'une quelconque des revendications précédentes, **caractérisé en ce que** le ressort de déversement (11) est un ressort de compression, en particulier un ressort hélicoïdal agissant en tant que ressort de compression.

7. Dispositif d'entraînement et de dosage selon l'une quelconque des revendications précédentes, **caractérisé en ce que** l'organe de propulsion (8) est retenu axialement au moyen d'un dispositif de préhension, qui vient en prise de préférence dans un filetage extérieur (8a) de l'organe de propulsion (8) axialement coulissant et solidaire en rotation par rapport au boîtier (4), dans lequel la mise en prise du dispositif de préhension dans l'organe de propulsion (8) est amovible par actionnement d'un organe d'actionnement (7), ce par quoi l'organe de propulsion (8) est coulissant dans la direction distale ou direction de déversement.

8. Dispositif d'entraînement et de dosage selon l'une quelconque des revendications précédentes, **caractérisé par** une douille de dosage (13), laquelle vient en prise dans un filetage extérieur (8a) de l'organe de propulsion (8) et peut être vissée par rotation d'un organe de dosage (3) par rapport à l'organe de propulsion (8), dans lequel une distance de dosage entre la douille de dosage (13) et une butée de dosage (4k) est augmentée par rotation de l'organe de dosage (3) dans un sens de rotation, laquelle entraîne une augmentation de dose.

9. Dispositif d'entraînement et de dosage selon la revendication précédente, **caractérisé en ce que** la distance entre la butée de dosage (4k) et la douille de dosage (13) est réduite par rotation de l'organe de dosage (3) dans un sens de rotation, laquelle entraîne une réduction de dose.

10. Dispositif d'entraînement et de dosage selon l'une quelconque des deux revendications précédentes, **caractérisé en ce que** la distance entre la douille de dosage (13) et la butée de dosage (4k) correspond à la course de déversement totale (H_{g}) de l'organe de propulsion (8).

11. Dispositif d'entraînement et de dosage selon la revendication précédente, **caractérisé en ce que** l'organe d'actionnement (7) s'enclenche lors de son actionnement avec l'organe de dosage (3), dans lequel cette liaison d'enclenchement est amovible au moyen de l'élément d'affichage de dose (10).

12. Dispositif d'entraînement et de dosage selon la revendication 7, **caractérisé en ce que** le dispositif de préhension comprend un organe de mise en prise de retenue (4h), lequel est formé par le boîtier (4) ou un anneau de préhension (17).
